# EUROPEAN PATENT APPLICATION

(11) **EP 4 723 845 A1**
(43) Date of publication of application: **08.04.2026**
(21) Application number: 24815215.9
(22) Date of filing: 15.05.2024
(51) Int. Cl.: H10K 30/60, H01L 27/146, H10K 30/85, H10K 39/32, H10K 85/60

(54) **COMPOUND, ORGANIC THIN FILM, PHOTOELECTRIC CONVERSION ELEMENT, IMAGING ELEMENT, PHOTOSENSOR, AND SOLID-STATE IMAGING DEVICE**

(30) Priority: 29.05.2023 JP 2023088105
(71) Applicant: MITSUBISHI GAS CHEMICAL COMPANY, INC., Chiyoda-ku Tokyo 100-8324 (JP)
(72) Inventor: SHIMIZU, Masahiro, Tokyo 125-8601 (JP); AOKI, Toshinari, Tokyo 125-8601 (JP); TAKAMURA, Tatsuro, Tokyo 125-8601 (JP); YAMAMOTO, Takashi, Tokyo 125-8601 (JP)
(74) Representative: Vossius & Partner Patentanwälte Rechtsanwälte mbB
(86) International application number: PCT/JP2024/018032
(87) International publication number: WO 2024/247731

(57) **Abstract**

Provided is a compound represented by the following formula (I): 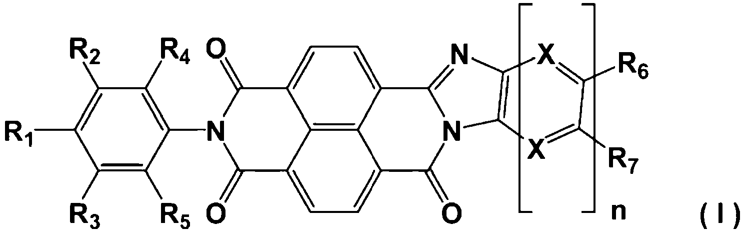 wherein X is each independently a methine group or a nitrogen atom; n is an integer of 0 or more and 3 or less; R₁ to R₅ are each independently a hydrogen atom, a halogen atom, a cyano group, a linear, branched or cyclic alkyl group, or the like; R₆ to R₇ are each independently a hydrogen atom, a halogen atom excluding a fluorine atom, a cyano group, a linear, branched or cyclic alkyl group, or the like; and one or more of R₁ to R₅ and one or more of R₆ to R₇ are each a halogen atom or a monovalent organic group having a Hammett substituent constant σp of 0.10 or more.

## Description

### Technical Field

The present disclosure relates to a compound, an organic thin film, a photoelectric conversion element, an image sensor, a photosensor and a solid-state imaging apparatus.

### Background Art

Techniques of converting visible light to electric signals by photoelectric conversion have heretofore been known and are used in, for example, image sensors. Such image sensors are carried by solid-state imaging apparatuses such as CCD (charge coupled device) image sensors and CMOS (complementary metal oxide semiconductor) image sensors. In recent years, reduction in pixel size have been underway in solid-state imaging apparatuses, and organic photoelectric conversion films adapted therefor have been studied. For example, Patent Documents 1 and 2 each disclose an organic photoelectric conversion film constituted by subphthalocyanine and an imide.

Solid-state imaging apparatuses are also required to achieve both high spectral selectivity and a high S/N ratio. Hence, the solid-state imaging apparatuses are desired to have high external quantum efficiency (EQE) and low dark current characteristics. An approach of disposing an electron transport layer and a hole blocking layer, and/or a hole transport layer and an electron blocking layer between a photoelectric conversion unit and an electrode unit is known in order to achieve both of such factors. In this context, each of the electron transport layer, the hole blocking layer and the electron blocking layer, etc. widely used in the field of organic electronic devices is disposed at the interface between an electrode or a film having conductivity and other films in films constituting a device. These layers play a role in controlling the back transfer of holes or electrons and adjusting the leakage of unnecessary holes or electrons. For example, Patent Document 3 discloses an example using 1,4,5,8-naphthalene-tetracarboxylic dianhydride (NTCDA) as a material for use in such a layer.

### Citation List

### Patent Document

Patent Documant1: Japanese Patent Application Laid-Open No. 2018-32754
Patent Document 2: Japanese Translation of PCT International Application Publication No. 2018-512423
Patent Document 3: Japanese Translation of PCT International Application Publication No. 2014-506736

### Summary of Invention

### Technical Problem

However, conventional hole blocking layers and electron blocking layers, including those disclosed in Patent Document 3, still have room for improvement not only in suppressing leak current in the dark but also in exhibiting high wavelength selectivity.

An object of the present invention is to provide a novel compound that is useful, particularly, in a photoelectric conversion element material, and a photoelectric conversion element material as well as an organic thin film, a photoelectric conversion element, an image sensor, a photosensor and a solid-state imaging apparatus including the compound. Another object of the present invention is to provide an image sensor, a photosensor, and a solid-state imaging apparatus that can suppress leak current in the dark and have excellent wavelength selectivity.

### Solution to Problem

The present invention is as described below.
[1] A compound represented by the following formula (I): 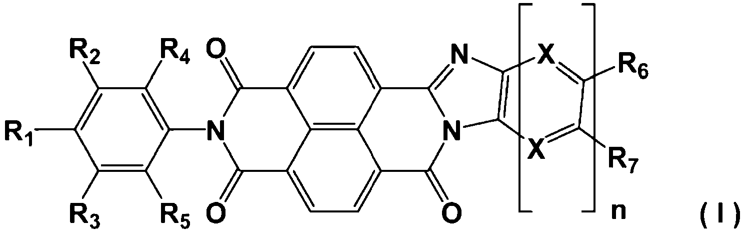 wherein
   X is each independently selected from the group consisting of a methine group and a nitrogen atom;
   n is an integer of 0 or more and 3 or less;
   R₁, R₂, R₃, R₄, and R₅ are each independently a hydrogen atom, a halogen atom, or a monovalent organic group, and one or more of R₁, R₂, R₃, R₄, and R₅ is a halogen atom or a monovalent organic group having a Hammett substituent constant σp of 0.10 or more;
   R₆ and R₇ are each independently a hydrogen atom, a halogen atom excluding a fluorine atom, or a monovalent organic group, and one or more of R₆ and R₇ is a halogen atom or a monovalent organic group having a Hammett substituent constant σp of 0.10 or more; and
   any adjacent members among R₁, R₂, R₃, R₄, R₅, R₆, and R₇ optionally constitute a portion of a condensed aliphatic ring or a condensed aromatic ring, and the condensed aliphatic ring and the condensed aromatic ring each optionally comprise one or more atoms other than a carbon atom.
[2] The compound according to [1], wherein
   R₁, R₂, R₃, R₄, and R₅ are each independently selected from the group consisting of a hydrogen atom, a halogen atom, a hydroxy group, a thiol group, an amino group, a cyano group, a carboxy group, a nitro group, and an optionally substituted linear, branched or cyclic alkyl group, thioalkyl group, thioaryl group, arylsulfonyl group, aryloxy group, alkylsulfonyl group, alkylamino group, arylamino group, alkoxy group, acylamino group, acyloxy group, aryl group, carboxyamide group, carboalkoxy group, carboaryloxy group, acyl group, and monovalent heterocyclic group, and
   R₆ and R₇ are each independently selected from the group consisting of a hydrogen atom, a halogen atom excluding a fluorine atom, a hydroxy group, a thiol group, an amino group, a cyano group, a carboxy group, a nitro group, and an optionally substituted linear, branched or cyclic alkyl group, thioalkyl group, thioaryl group, arylsulfonyl group, aryloxy group, alkylsulfonyl group, alkylamino group, arylamino group, alkoxy group, acylamino group, acyloxy group, aryl group, carboxyamide group, carboalkoxy group, carboaryloxy group, acyl group, and monovalent heterocyclic group.
[3] The compound according to [1] or [2], wherein R₁ is a cyano group.
[4] The compound according to any of [1] to [3], wherein R₄ and R₅ are hydrogen atoms.
[5] The compound according to any of [1] to [4], wherein n is 0 and R₆ and R₇ are cyano groups.
[6] A compound represented by the following formula (V): 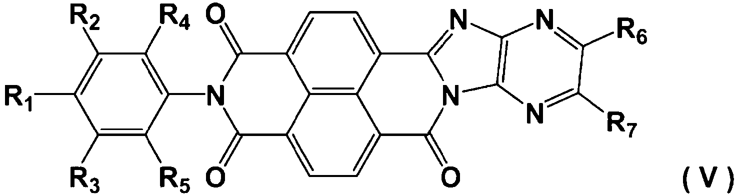 wherein
   R₁, R₂, R₃, R₄, and R₅ are each independently selected from the group consisting of a hydrogen atom, a halogen atom, a hydroxy group, a thiol group, an amino group, a cyano group, a carboxy group, a nitro group, and an optionally substituted linear, branched or cyclic alkyl group, thioalkyl group, thioaryl group, arylsulfonyl group, aryloxy group, alkylsulfonyl group, alkylamino group, arylamino group, alkoxy group, acylamino group, acyloxy group, aryl group, carboxyamide group, carboalkoxy group, carboaryloxy group, acyl group, and monovalent heterocyclic group, and one or more of R₁, R₂, R₃, R₄, and R₅ is a hydrogen atom, or a halogen atom or a monovalent organic group having a Hammett substituent constant σp of 0.10 or more; and
   R₆ and R₇ are each independently a hydrogen atom, a halogen atom, or a monovalent organic group, one or more of R₆ and R₇ is a halogen atom or a monovalent organic group having a Hammett substituent constant σp of 0.10 or more, any adjacent members among R₁, R₂, R₃, R₄, R₅, R₆, and R₇ optionally constitute a portion of a condensed aliphatic ring or a condensed aromatic ring, and the condensed aliphatic ring and the condensed aromatic ring each optionally comprise one or more atoms other than a carbon atom.
[7] The compound according to any of [1] to [6], wherein an energy level of the lowest unoccupied molecular orbital obtained by density functional formalism is -6.00 eV or more and -3.60 eV or less.
[8] The compound according to any of [1] to [7], wherein a difference between an energy level of the lowest unoccupied molecular orbital and an energy level of the highest occupied molecular orbital obtained by density functional formalism is 2.85 eV or more and 4.00 eV or less.
[9] The compound according to any of [1] to [8], wherein a 5% mass reduction temperature measured under the following Condition 1 is 430°C or higher and 550°C or lower:
   (Condition 1)
   Pressure: 101325 Pa
   Nitrogen: 200 mL/min
   Temperature increase condition: increased from 20°C to 550°C at 10°C/min.
[10] The compound according to any of [1] to [9], wherein a 10% mass reduction temperature measured under the following Condition 2 is 200°C or higher and 400°C or lower:
   (Condition 2)
   Pressure: 1 Pa
   Temperature increase condition: increased from 20°C to 450°C at 10°C/min.
[11] The compound according to any of [1] to [10], wherein the compound is a material for a photoelectric conversion element.
[12] An organic thin film comprising the compound according to any of [1] to [10].
[13] A photoelectric conversion element comprising a first electrode film, a second electrode film, and a photoelectric conversion film positioned between the first electrode film and the second electrode film, wherein
   the photoelectric conversion film comprises the material for a photoelectric conversion element according to [11].
[14] A photoelectric conversion element comprising a first electrode film, a second electrode film, and a photoelectric conversion film positioned between the first electrode film and the second electrode film, wherein
   the photoelectric conversion film comprises the organic thin film according to [12].
[15] The photoelectric conversion element according to [14], wherein
   the photoelectric conversion film comprises a photoelectric conversion layer and an auxiliary layer, wherein
   the auxiliary layer is made of only the organic thin film or made of a plurality of films comprising the organic thin film.
[16] A photoelectric conversion element comprising a first electrode film, a second electrode film, and a photoelectric conversion film positioned between the first electrode film and the second electrode film, wherein
   the photoelectric conversion film comprises a photoelectric conversion layer and two auxiliary layers positioned between the photoelectric conversion layer and the second electrode film, wherein
   among the two auxiliary layers, an auxiliary layer closer to the second electrode film comprises the organic thin film according to [12].
[17] An image sensor comprising the photoelectric conversion element according to any of [13] to [16].
[18] The image sensor according to [17], wherein the image sensor is prepared by laminating two or more photoelectric conversion elements.
[19] An image sensor prepared by disposing a plurality of photoelectric conversion elements according to any of [13] to [16] in an array pattern.
[20] A photosensor comprising the image sensor according to any of [17] to [19].
[21] A solid-state imaging apparatus comprising the image sensor according to any of [17] to [19].
[22] An image sensor comprising the compound represented by the following formula (VI): 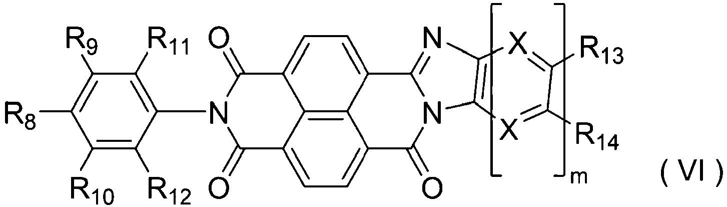 wherein
   X is each independently selected from the group consisting of a methine group and a nitrogen atom;
   m is an integer of 0 or more and 3 or less;
   R₈, R₉, R₁₀, R₁₁, and R₁₂ are each independently a hydrogen atom, a halogen atom having a Hammett substituent constant σp of less than 0.10, or a monovalent organic group having a Hammett substituent constant σp of less than 0.10;
   R₁₃ and R₁₄ are each independently a hydrogen atom, a halogen atom, or a monovalent organic group, and one or more of R₁₃ and R₁₄ is a halogen atom or a monovalent organic group having a Hammett substituent constant σp of 0.10 or more; and
   any adjacent members among R₈, R₉, R₁₀, R₁₁, R₁₂, R₁₃, and R₁₄ optionally constitute a portion of a condensed aliphatic ring or a condensed aromatic ring, and the condensed aliphatic ring and the condensed aromatic ring each optionally comprise one or more atoms other than a carbon atom.
[23] An image sensor comprising a photoelectric conversion element, wherein
   the photoelectric conversion element comprises a first electrode film, a second electrode film, and a photoelectric conversion film positioned between the first electrode film and the second electrode film, wherein
   the photoelectric conversion film comprises a compound represented by the following formula (VI): 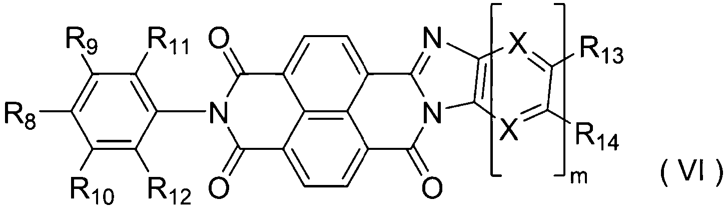
   wherein
   X is each independently selected from the group consisting of a methine group and a nitrogen atom;
   m is an integer of 0 or more and 3 or less;
   R₈, R₉, R₁₀, R₁₁, and R₁₂ are each independently a hydrogen atom, a halogen atom having a Hammett substituent constant σp of less than 0.10, or a monovalent organic group having a Hammett substituent constant σp of less than 0.10;
   R₁₃ and R₁₄ are each independently a hydrogen atom, a halogen atom, or a monovalent organic group, and one or more of R₁₃ and R₁₄ is a halogen atom or a monovalent organic group having a Hammett substituent constant σp of 0.10 or more; and
   any adjacent members among R₈, R₉, R₁₀, R₁₁, R₁₂, R₁₃, and R₁₄ optionally constitute a portion of a condensed aliphatic ring or a condensed aromatic ring, and the condensed aliphatic ring and the condensed aromatic ring each optionally comprise one or more atoms other than a carbon atom.
[24] An image sensor comprising a photoelectric conversion element, wherein
   the photoelectric conversion element comprises a first electrode film, a second electrode film, and a photoelectric conversion film positioned between the first electrode film and the second electrode film, wherein
   the photoelectric conversion film comprises a photoelectric conversion layer and two auxiliary layers positioned between the photoelectric conversion layer and the second electrode film, wherein
   among the two auxiliary layers, an auxiliary layer closer to the second electrode film comprises a compound represented by the following formula (VI): 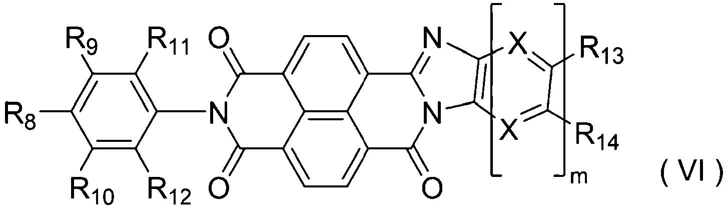
   wherein
   X is each independently selected from the group consisting of a methine group and a nitrogen atom;
   m is an integer of 0 or more and 3 or less;
   R₈, R₉, R₁₀, R₁₁, and R₁₂ are each independently a hydrogen atom, a halogen atom having a Hammett substituent constant σp of less than 0.10, or a monovalent organic group having a Hammett substituent constant σp of less than 0.10;
   R₁₃ and R₁₄ are each independently a hydrogen atom, a halogen atom, or a monovalent organic group, and one or more of R₁₃ and R₁₄ is a halogen atom or a monovalent organic group having a Hammett substituent constant σp of 0.10 or more; and
   any adjacent members among R₈, R₉, R₁₀, R₁₁, R₁₂, R₁₃, and R₁₄ optionally constitute a portion of a condensed aliphatic ring or a condensed aromatic ring, and the condensed aliphatic ring and the condensed aromatic ring each optionally comprise one or more atoms other than a carbon atom.
[25] The image sensor according to [23] or [24], wherein the image sensor is prepared by laminating two or more photoelectric conversion elements.
[26] The image sensor according to any of [23] to [25], wherein the image sensor is prepared by disposing a plurality of photoelectric conversion elements in an array pattern.
[27] A photosensor comprising the image sensor according to any of [22] to [26].
[28] A solid-state imaging apparatus comprising the image sensor according to any of [22] to [26].

### Advantageous Effect of Invention

The present invention can provide a novel compound useful, particularly, in a photoelectric conversion element material, a photoelectric conversion element material, and an organic thin film, a photoelectric conversion element, an image sensor, a photosensor and a solid-state imaging apparatus comprising the compound. The present invention can also provide an image sensor, a photosensor, and a solid-state imaging apparatus that can suppress leak current in the dark and have excellent wavelength selectivity.

### Brief Description of Drawings

[Figure 1] Figure 1 is a cross-sectional schematic view partially showing one example of the photoelectric conversion element of the present invention.
[Figure 2] Figure 2 is a cross-sectional schematic view partially showing another example of the photoelectric conversion element of the present invention.

### Description of Embodiments

Hereinafter, the mode for carrying out the present invention (hereinafter, simply referred to as the "present embodiment") will be described in detail with reference to the drawings, if necessary. However, the present invention is not limited by the present embodiment described below. Various changes or modifications can be made in the present invention without departing from the spirit of the present invention. In the drawings, the same numerals or symbols will be used to designate the same components, so that the description will be omitted. Positional relationship indicated by terms such as "up", "down", "right" and "left" is based on the positional relationship shown in the drawings, unless otherwise specified. The dimensional ratios of the drawings are not limited to the shown ratios.

In the present specification, the Hammett substituent constant σp is represented by log(K/K₀), wherein K and K₀ are the dissociation constants of para-substituted and unsubstituted benzoic acid in water at 25°C. The Hammett substituent constants σp of atoms and organic groups are described in, for example, Journal of Synthetic Organic Chemistry, Japan, The Society of Synthetic Organic Chemistry, Japan, Vol. 23, No. 8 (1965). Among them, the Hammett substituent constants σp of representative atoms and organic groups are as follows.

**[Table 1]**

| Atom or organic group | σp |
|---|---|
| H | 0.000 |
| CN | 0.660 |
| CF₃ | 0.54 |
| F | 0.062 |
| Cl | 0.227 |
| Br | 0.232 |
| NO₂ | 0.778 |
| CH₃ | -0.170 |
| OCH₃ | -0.268 |

In the present specification, a monovalent organic group refers to a monovalent group made of one or more of carbon atoms, hydrogen atoms, nitrogen atoms, oxygen atoms, and sulfur atoms.

In the present specification, examples of the halogen atom include a fluorine atom (F), a chlorine atom (CI), a bromine atom (Br) and an iodine atom (I).

In the present specification, the linear alkyl group may be a linear alkyl group in which the number of carbon atoms in the alkyl group is 1 to 12. Examples thereof include a methyl group (Me), an ethyl group (Et), a n-propyl group (n-Pr), a n-butyl group (n-Bu), a n-pentyl group, a n-hexyl group, a n-heptyl group, a n-octyl group, a n-nonyl group, a n-decyl group, and a n-dodecyl group.

In the present specification, the branched alkyl group may be a branched alkyl group in which the number of carbon atoms in the alkyl group is 1 to 12. Examples thereof include an isopropyl group (i-Pr), a sec-butyl group (s-Bu), a tert-butyl group (t-Bu), an isopentyl group, a sec-pentyl group, a 3-pentyl group, a neopentyl group, an isohexyl group, an isooctyl group, an isononyl group, an isodecyl group and isododecyl group. The linear or branched alkyl group may have a substituent. Examples of the substituent include halogen atoms such as a fluorine atom, monovalent groups having an aromatic ring such as a benzyl group, a naphthyl group and a phenoxy group, monovalent groups having a heteroatom such as alkoxy groups, aminoalkyl and thioalkyl groups, monovalent groups having a heterocyclic ring such as a pyridyl group, a hydroxy group, a carboxyl group, an amino group, and a thiol group.

In the present specification, the cyclic alkyl group may be a cyclic alkyl group in which the number of carbon atoms in the alkyl group is 3 to 10. Examples thereof include a cyclopropyl group, a cyclobutyl group, a cyclopentyl group, a cyclohexyl group, a cycloheptyl group and a cyclooctyl group. The cyclic alkyl group may have a heteroatom such as a nitrogen atom, an oxygen atom, and/or a sulfur atom in its ring. Examples of such a cyclic alkyl group include a pyrrolidinyl group, an oxazolidinyl group, a pyrazolidinyl group, a thiazolidinyl group, an imidazolidinyl group, a dioxofuranyl group, a tetrahydrofuranyl group, a tetrahydrothiophenyl group, a piperazinyl group, a dioxanyl group, and a morpholinyl group. A monovalent group such as a hydroxy group, a carboxyl group, an amino group and/or a thiol group may be further bonded to the cyclic alkyl group.

In the present specification, the thioalkyl group (-SR; hereinafter, R represents an alkyl group) and the thioaryl group (-SAr; hereinafter, Ar represents an aryl group) may be a thioalkyl group in which the number of carbon atoms in the alkyl group is 1 to 12 and a thioaryl group in which the number of carbon atoms in the aryl group is 6 to 16. The thioalkyl group and the thioaryl group may further have a substituent including an amino group, a hydroxy group, halogen atoms, alkoxy groups, and thioalkyl groups. Examples of such a thioalkyl group and a thioaryl group include a methylthio group, an ethylthio group, a phenylthio group, a toluylthio group, an aminophenylthio group, a hydroxyphenylthio group, a fluorophenylthio group, a dimethylphenylthio group, and a methylthiophenylthio group.

In the present specification, the arylsulfonyl group (-SO₂-Ar) may be an arylsulfonyl group in which the number of carbon atoms in the aryl group is 6 to 16. Examples thereof include a phenylsulfonyl group, a toluenesulfonyl group, a dimethylbenzenesulfonyl group, a mesitylenesulfonyl group, an octylbenzenesulfonyl group, and a naphthalenesulfonyl group.

In the present specification, the aryloxy group (-O-Ar) may be an aryloxy group in which the number of carbon atoms in the aryl group is 6 to 16. The aryloxy group may further have a substituent including a cyano group, halogen atoms such as a fluorine atom, a hydroxy group, alkoxy groups such as a methoxy group, an amino group, alkylamino groups, a thiol group, and aryloxy groups. Examples of such an aryloxy group include a phenoxy group, a cyanophenoxy group, a methylcyanophenoxy group, a dimethylcyanophenoxy group, a fluorocyanophenoxy group, a dicyanophenoxy group, a methoxycyanophenoxy group, a tricyanophenoxy group, a cyanonaphthoxy group, a dicyanonaphthoxy group, a 2-methylphenoxy group, a 3-methylphenoxy group, a 4-methylphenoxy group, a fluoromethylphenoxy group, a dimethylphenoxy group, a 3-hydroxyphenoxy group, a fluoro-3-hydroxyphenoxy group, a 2-hydroxyphenoxy group, a fluoro-2-hydroxyphenoxy group, a methoxyphenoxy group, an ethoxyphenoxy group, a fluorophenoxy group, a perfluorophenoxy group, a dimethoxyphenoxy group, an aminophenoxy group, a N,N-dimethylaminophenoxy group, a thiophenoxy group, a (trifluoromethyl)phenoxy group, a naphthoxy group, a methoxynaphthoxy group, a fluoronaphthoxy group, and a phenoxyphenoxy group.

In the present specification, the alkylsulfonyl group (-SO₂-R) may be an alkylsulfonyl group in which the number of carbon atoms in the alkyl group is 1 to 12. Examples thereof include a mesyl group, an ethylsulfonyl group, and a n-butylsulfonyl group.

In the present specification, the alkylamino group (wherein the alkylamino group is -NHR or -NR₂ wherein two R moieties may be the same as or different from each other) may be an alkylamino group in which the number of carbon atoms in the alkyl group is 1 to 12. Examples thereof include a methylamino group, an ethylamino group, a n-propylamino group, a n-butylamino group, a n-pentylamino group, a n-hexylamino group, a n-heptylamino group, a n-octylamino group, a n-nonylamino group, a n-decylamino group, a n-dodecylamino group, an isopropylamino group, a sec-butylamino group, a tert-butylamino group, an isopentylamino group, a sec-pentylamino group, a 3-pentylamino group, a neopentylamino group, an isohexylamino group, an isoheptylamino group, an isooctylamino group, an isononylamino group, an isodecylamino group and isododecylamino group, a dimethylamino group, a diethylamino group, a diisopropylamino group, and an isopropylethylamino group.

In the present specification, the arylamino group (wherein the arylamino group is -NHAr or -NAr₂ wherein two Ar moieties may be the same as or different from each other) may be an arylamino group in which the number of carbon atoms in the aryl group is 6 to 16. Examples thereof include an anilino group, a toluidinyl group, a dimethylanilino group, an isopropylanilino group, a t-butylanilino group, a fluoroanilino group, a trifluoromethylanilino group, a bis(trifluoromethyl)anilino group, a pyridylamino group, a methylpyridylamino group, a fluoropyridylamino group, a pyrimidylamino group, and a biphenylamino group.

In the present specification, the alkoxy group (-OR) may be an alkoxy group having 1 to 12 carbon atoms. Examples thereof include a methoxy group, an ethoxy group, a n-propoxy group, a n-butyloxy group, a n-pentoxy group, a n-hexoxy group, a n-heptoxy group, a n-octoxy group, a n-nonoxy group, a n-decoxy group and n-dodecoxy group, an isopropoxy group, a sec-butyloxy group, a tert-butyloxy group, an isopentoxy group, a sec-pentoxy group, a 3-pentoxy group, a neopentoxy group, an isohexoxy group, an isooctoxy group, an isononoxy group, an isodecoxy group, and an isododecoxy group.

In the present specification, the acylamino group (-NH-COR or -NH-COAr) may be a group in which the number of carbon atoms in the alkyl group is 1 to 12 or the number of carbon atoms in the aryl group is 6 to 16, and may have a substituent including halogen atoms such as a fluorine atom, alkoxy groups, and a cyano group. Examples of such an acylamino group include an acetylamino group, a propionylamino group, a benzoylamino group, a methylbenzoylamino group, a dimethylbenzoylamino group, a methoxybenzoylamino group, a cyanobenzoylamino group, and a bis(trifluoromethyl)benzoylamino group.

In the present specification, the acyloxy group (-O-COR or -O-COAr) may be a group in which the number of carbon atoms in the alkyl group is 1 to 12 or the number of carbon atoms in the aryl group is 6 to 16. The acyloxy group may further have a substituent including halogen atoms such as a fluorine atom, and a cyano group and may have a heteroatom such as a nitrogen atom in an aromatic ring. Examples of such an acyloxy group include a benzoyloxy group, a toluoyloxy group, a dimethylbenzoyloxy group, a cyanobenzoyloxy group, a fluorobenzoyloxy group, a bis(trifluoromethyl)benzoyloxy group, a pyridinecarboxy group, and a methylpyridinecarboxy group.

In the present specification, the aryl group (-Ar) may be an aryl group having 6 to 16 carbon atoms. The aryl group may further have a substituent including an amino group, a hydroxy group, a thiol group, halogen atoms such as a fluorine atom, a nitro group, and a cyano group and may have a heteroatom such as a nitrogen atom in an aromatic ring. Examples of such an aryl group include a phenyl group, a methylphenyl group, an ethylphenyl group, a dimethylphenyl group, a trimethylphenyl group, a methoxyphenyl group, a dimethoxyphenyl group, a trimethoxyphenyl group, a methoxymethylphenyl group, an aminophenyl group, a diaminophenyl group, an aminomethylphenyl group, a hydroxyphenyl group, a dihydroxyphenyl group, a hydroxymethylphenyl group, a hydroxyethylphenyl group, a thiophenyl group, a methylthiophenyl group, a dithiophenyl group, a fluorophenyl group, a fluoromethylphenyl group, a trifluoromethylphenyl group, a perfluorophenyl group, a fluoro(trifluoromethyl)phenyl group, a bis(trifluoromethyl)phenyl group, a cyanophenyl group, a methylcyanophenyl group, a dimethylcyanophenyl group, a dicyanophenyl group, a methoxycyanophenyl group, a tricyanophenyl group, a dicyanophenyl group, a methylcyanopyridyl group, a (trifluoromethyl)cyanopyridyl group, a dimethylcyanopyridyl group, a dicyanopyridyl group, a methoxycyanopyridyl group, a tricyanopyridyl group, a cyanopyridyl group, a naphthyl group, a nitrophenyl group, a dinitrophenyl group, a nitrofluorophenyl group, a methylnaphthyl group, an ethylnaphthyl group, a dimethylnaphthyl group, a trimethylnaphthyl group, a methoxynaphthyl group, a dimethoxynaphthyl group, a trimethoxynaphthyl group, an aminonaphthyl group, a diaminonaphthyl group, an aminomethylnaphthyl group, a hydroxynaphthyl group, a dihydroxynaphthyl group, a hydroxymethylnaphthyl group, a hydroxyethylnaphthyl group, a thionaphthyl group, a methylthionaphthyl group, a dithionaphthyl group, a fluoronaphthyl group, a trifluoromethylnaphthyl group, a perfluoronaphthyl group, a di(trifluoromethyl)naphthyl group, a biphenyl group, and a cyanobiphenyl group.

In the present specification, the carboxyamide group (wherein the carboxyamide group is -CO-NH₂, -CO-NHR, -CONR₂ wherein two R moieties may be the same as or different from each other, -CONHAr, or -CONAr₂ wherein two Ar may be the same as or different from each other) may be a carboxyamide group in which the number of carbon atoms in the alkyl group is 1 to 12 or the number of carbon atoms in the aryl group is 6 to 16. Examples thereof include a dimethylcarboxyamide group and a diphenylcarboxyamide group.

In the present specification, the carboalkoxy group or the carboaryloxy group (-COOR or -COOAr) may be a carboalkoxy group in which the number of carbon atoms in the alkyl group is 1 to 12 or the number of carbon atoms in the aryl group is 6 to 16. Examples thereof include a carbomethoxy group and a carbophenoxy group.

In the present specification, the monovalent heterocyclic group may be a monovalent heterocyclic group having 3 to 14 carbon atoms. Examples thereof include a furanyl group, a thienyl group, a pyrrolyl group, a pyrazolyl group, an imidazolyl group, a triazolyl group, an oxazolyl group, a dioxazolyl group, an isoxazolyl group, an oxadiazolyl group, a thiazolyl group, an isothiazolyl group, a thiadiazolyl group, a triazolyl group, an indolyl group, an indolinyl group, an indolizinyl group, an indazolinyl group, indoleninyl group, a benzofuranyl group, a benzothienyl group, a carbazolyl group, a dibenzofuranyl group, a dibenzothienyl group, a pyridinyl group, a diazinyl group, an oxazinyl group, a thiazinyl group, a dioxinyl group, a dithienyl group, a triazinyl group, a pyrimidinyl group, a pyrazinyl group, a pyridazinyl group, a quinolinyl group, an isoquinolinyl group, a cinnolinyl group, a phthalazinyl group, a quinazolinyl group, a naphthyridinyl group, a purinyl group, a pteridinyl group, an acridinyl group, a phenanthridinyl group, a phenanthrolinyl group, a xanthenyl group, a phenoxazinyl group, a thianthrenyl group, a morpholinyl group and a phenazinyl group.

### (Compound (I))

The compound according to one aspect of the present embodiment is represented by the following formula (I) (hereinafter, this compound is also referred to as a "compound (I)"): 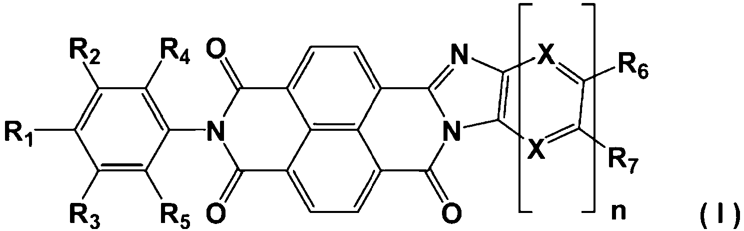 In the formula, X is each independently selected from the group consisting of a methine group (CH group) and a nitrogen atom; n is an integer of 0 or more and 3 or less; and R₁, R₂, R₃, R₄, and R₅ are each independently a hydrogen atom, a halogen atom, or a monovalent organic group, and one or more of R₁, R₂, R₃, R₄, and R₅ is a halogen atom or a monovalent organic group having a Hammett substituent constant σp of 0.10 or more. Also, R₆ and R₇ are each independently a hydrogen atom, a halogen atom excluding a fluorine atom, or a monovalent organic group, and one or more of R₆ and R₇ is a halogen atom or a monovalent organic group having a Hammett substituent constant σp of 0.10 or more.

R₁, R₂, R₃, R₄, and R₅ may be each independently selected from the group consisting of a hydrogen atom, a halogen atom, a hydroxy group, a thiol group, an amino group, a cyano group, a carboxy group, a nitro group, and an optionally substituted linear, branched or cyclic alkyl group, thioalkyl group, thioaryl group, arylsulfonyl group, aryloxy group, alkylsulfonyl group, alkylamino group, arylamino group, alkoxy group, acylamino group, acyloxy group, aryl group, carboxyamide group, carboalkoxy group, carboaryloxy group, acyl group, and monovalent heterocyclic group, and R₆ and R₇ may be each independently selected from the group consisting of a hydrogen atom, a halogen atom excluding a fluorine atom, a hydroxy group, a thiol group, an amino group, a cyano group, a carboxy group, a nitro group, and an optionally substituted linear, branched or cyclic alkyl group, thioalkyl group, thioaryl group, arylsulfonyl group, aryloxy group, alkylsulfonyl group, alkylamino group, arylamino group, alkoxy group, acylamino group, acyloxy group, aryl group, carboxyamide group, carboalkoxy group, carboaryloxy group, acyl group, and monovalent heterocyclic group. Any adjacent members among R₁, R₂, R₃, R₄, R₅, R₆, and R₇ optionally constitute a portion of a condensed aliphatic ring or a condensed aromatic ring, and the condensed aliphatic ring and the condensed aromatic ring each optionally contain one or more atoms other than a carbon atom. Compounds represented by the following formula (II), formula (III), and formula (IV) are excluded from the compound (I).

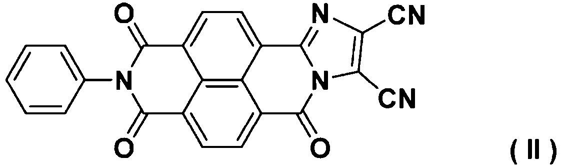

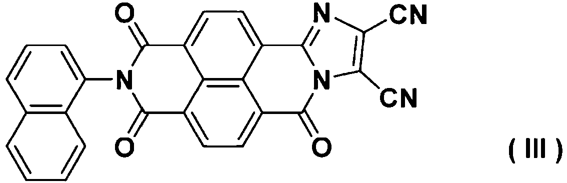

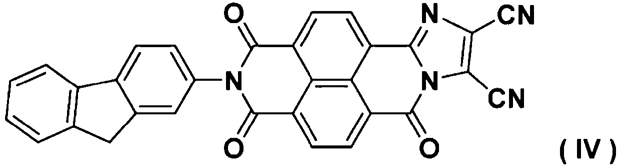

Such a compound (I) can suppress leak current in the dark and has excellent wavelength selectivity, and exhibits excellent characteristics, particularly, as a photoelectric conversion element material. A factor for this is not certain and is considered as follows by the present inventors, though the factor is not limited thereto: that is, when R₁, R₂, R₃, R₄, R₅, R₆, and R₇ have the above-described Hammett substituent constants σp and a structure with an extended π-conjugated system, the energy level of the lowest unoccupied molecular orbital of the compound (I) is lowered, enabling suppression of leak current in the dark and providing excellent wavelength selectivity.

From such a viewpoint, it is preferable that one or two of R₁, R₂, R₃, R₄, and R₅ have Hammett substituent constants σp of 0.10 or more. Moreover, one or two of R₁, R₂, R₃, R₄, and R₅ may have Hammett substituent constants σp of 0.10 or more, and the remaining may be hydrogen atoms. From the same viewpoint, it is more preferable that one or two of R₁, R₂, R₃, R₄, and R₅ have Hammett substituent constants σp of 0.20 or more, and further preferable that they have σp of 0.30 or more. The upper limit of the Hammett substituent constants σp may be, without particular limitations, 2.0, 3.0, or 4.0.

From the same viewpoint, it is preferable that both R₆ and R₇ have Hammett substituent constants σp of 0.10 or more, more preferable that they have σp of 0.20 or more, and further preferable that they have σp of 0.30 or more. The upper limit of the Hammett substituent constants σp may be, without particular limitations, 2.0, 3.0, or 4.0.

Examples of halogen atoms and monovalent groups having Hammett substituent constants σp of 0.10 or more include a chlorine atom, a bromine atom, an iodine atom, a cyano group, a trifluoromethyl group, and a nitro group.

In the compound (I) of the present embodiment, without particular limitations, X is more preferably a nitrogen atom from the viewpoint of more effectively and reliably exerting the working effects according to the present invention. Moreover, in the compound (I), without particular limitations, the two X moieties may be the same as or different from each other and are preferably the same as each other.

In the compound (I) of the present embodiment, n is not particularly limited, but it is preferable that n is an integer of 0 or more and 2 or less, and more preferable that n is 0 or 1.

In the compound (I) of the present embodiment, from the viewpoint of more effectively and reliably exerting the working effects according to the present invention, without particular limitations, R₁, R₂, R₃, R₄, and R₅ are preferably each independently selected from the group consisting of a hydrogen atom, a halogen atom, a hydroxy group, a thiol group, an amino group, a cyano group, a carboxy group, a nitro group, and an optionally substituted linear, branched or cyclic alkyl group, thioalkyl group, thioaryl group, arylsulfonyl group, aryloxy group, alkylsulfonyl group, alkylamino group, arylamino group, alkoxy group, acylamino group, acyloxy group, aryl group, carboxyamide group, carboalkoxy group, carboaryloxy group, acyl group, and monovalent heterocyclic group, more preferably each independently selected from the group consisting of a hydrogen atom, a halogen atom, a nitro group, a cyano group, and an optionally substituted linear, branched or cyclic alkyl group, and particularly preferably each independently selected from the group consisting of a hydrogen atom, a halogen atom, a nitro group, a cyano group, and a halogen atom-substituted alkyl group. The compound (I) having the structure mentioned above can further suppress leak current in the dark and tends to have better wavelength selectivity.

In the compound (I) of the present embodiment, R₁, R₂, R₃, R₄ and R₅ may be the same or different. Without particular limitations, two moieties selected from R₁, R₂, R₃, R₄ and R₅ are preferably the same, and at least three moieties selected from R₁, R₂, R₃, R₄ and R₅ are more preferably the same, from the viewpoint of more effectively and reliably exerting the working effects according to the present invention.

In the compound (I) of the present embodiment, from the viewpoint of more effectively and reliably exerting the working effects according to the present invention, without particular limitations, at least one of R₁, R₂, R₃, R₄, and R₅ is preferably not a hydrogen atom; at least one of R₁, R₂, R₃, R₄, and R₅ is more preferably selected from the group consisting of a halogen atom, a nitro group, a cyano group, and an optionally substituted linear, branched or cyclic alkyl group; and at least one of R₁, R₂, R₃, R₄, and R₅ is particularly preferably selected from the group consisting of a halogen atom, a nitro group, a cyano group, and a halogen atom-substituted alkyl group. Moreover, at least R₁ is preferably selected from the group consisting of a halogen atom, a nitro group, a cyano group, and a halogen atom-substituted alkyl group, and is more preferably a cyano group.

In the compound (I) of the present embodiment, without particular limitations, at least two of R₁, R₂, R₃, R₄ and R₅ are preferably hydrogen atoms, at least two or at least three of R₁, R₂, R₃, R₄ and R₅ are more preferably hydrogen atoms, and R₄ and R₅ are particularly preferably hydrogen atoms, from the viewpoint of more effectively and reliably exerting the working effects according to the present invention.

In the compound (I) of the present embodiment, without particular limitations, R₆ and R₇ are preferably each independently selected from the group consisting of a hydrogen atom, a halogen atom excluding a fluorine atom, a hydroxy group, a thiol group, an amino group, a cyano group, a carboxy group, a nitro group, and an optionally substituted linear, branched or cyclic alkyl group, thioalkyl group, thioaryl group, arylsulfonyl group, aryloxy group, alkylsulfonyl group, alkylamino group, arylamino group, alkoxy group, acylamino group, acyloxy group, aryl group, carboxyamide group, carboalkoxy group, carboaryloxy group, acyl group, and monovalent heterocyclic group, more preferably each independently selected from the group consisting of a hydrogen atom, a chlorine atom, a bromine atom, an iodine atom, a nitro group, a cyano group, and an optionally substituted linear, branched or cyclic alkyl group, and particularly preferably each independently selected from the group consisting of a hydrogen atom, a chlorine atom, a bromine atom, an iodine atom, a nitro group, a cyano group, and a halogen atom-substituted alkyl group. The compound (I) having the structure mentioned above can further suppress leak current in the dark and tends to have better wavelength selectivity. Moreover, the 5% mass reduction temperature or 10% mass reduction temperature described later tends to be higher, and manufacturability tends to be better.

In the compound (I) of the present embodiment, R₆ and R₇ may be the same or different.

Hereinafter, preferred combinations of R₁, R₂, R₃, R₄, R₅, R₆, and R₇ in the compound (I) will be shown.

| R₁ | R₂ | R₃ | R₄ | R₅ | R₆ | R₇ |
|---|---|---|---|---|---|---|
| H | H | H | H | H | H | CN |
| CN | H | H | H | H | H | CN |
| CN | CN | H | H | H | H | CN |
| CN | H | H | CN | H | H | CN |
| CN | H | H | CN | CN | H | CN |
| CN | H | H | CN | CF₃ | H | CN |
| CF₃ | H | H | H | H | H | CN |
| CF₃ | CF₃ | H | H | H | H | CN |
| CF₃ | H | H | CF₃ | H | H | CN |
| CF₃ | H | H | CF₃ | CF₃ | H | CN |
| CF₃ | H | H | CN | CF₃ | H | CN |
| CN | CF₃ | H | H | H | H | CN |
| CN | H | H | CF₃ | H | H | CN |
| CF₃ | CN | H | H | H | H | CN |
| H | H | H | H | H | CN | H |
| CN | H | H | H | H | CN | H |
| CN | CN | H | H | H | CN | H |
| CN | H | H | CN | H | CN | H |
| CN | H | H | CN | CN | CN | H |
| CN | H | H | CN | CF₃ | CN | H |
| CF₃ | H | H | H | H | CN | H |
| CF₃ | CF₃ | H | H | H | CN | H |
| CF₃ | H | H | CF₃ | H | CN | H |
| CF₃ | H | H | CF₃ | CF₃ | CN | H |
| CF₃ | H | H | CN | CF₃ | CN | H |
| CN | CF₃ | H | H | H | CN | H |
| CN | H | H | CF₃ | H | CN | H |
| CF₃ | CN | H | H | H | CN | H |
| H | H | H | H | H | CN | CN |
| CN | H | H | H | H | CN | CN |
| CN | CN | H | H | H | CN | CN |
| CN | H | H | CN | H | CN | CN |
| CN | H | H | CN | CN | CN | CN |
| CN | H | H | CN | CF₃ | CN | CN |
| CF₃ | H | H | H | H | CN | CN |
| CF₃ | CF₃ | H | H | H | CN | CN |
| CF₃ | H | H | CF₃ | H | CN | CN |
| CF₃ | H | H | CF₃ | CF₃ | CN | CN |
| CF₃ | H | H | CN | CF₃ | CN | CN |
| CN | CF₃ | H | H | H | CN | CN |
| CN | H | H | CF₃ | H | CN | CN |
| CF₃ | CN | H | H | H | CN | CN |
| H | H | H | H | H | H | Cl |
| CN | H | H | H | H | H | Cl |
| CN | CN | H | H | H | H | Cl |
| CN | H | H | CN | H | H | Cl |
| CN | H | H | CN | CN | H | Cl |
| CN | H | H | CN | CF₃ | H | Cl |
| CF₃ | H | H | H | H | H | Cl |
| CF₃ | CF₃ | H | H | H | H | Cl |
| CF₃ | H | H | CF₃ | H | H | Cl |
| CF₃ | H | H | CF₃ | CF₃ | H | Cl |
| CF₃ | H | H | CN | CF₃ | H | Cl |
| CN | CF₃ | H | H | H | H | Cl |
| CN | H | H | CF₃ | H | H | Cl |
| CF₃ | CN | H | H | H | H | Cl |
| H | H | H | H | H | Cl | H |
| CN | H | H | H | H | Cl | H |
| CN | CN | H | H | H | Cl | H |
| CN | H | H | CN | H | Cl | H |
| CN | H | H | CN | CN | Cl | H |
| CN | H | H | CN | CF₃ | Cl | H |
| CF₃ | H | H | H | H | Cl | H |
| CF₃ | CF₃ | H | H | H | Cl | H |
| CF₃ | H | H | CF₃ | H | Cl | H |
| CF₃ | H | H | CF₃ | CF₃ | Cl | H |
| CF₃ | H | H | CN | CF₃ | Cl | H |
| CN | CF₃ | H | H | H | Cl | H |
| CN | H | H | CF₃ | H | Cl | H |
| CF₃ | CN | H | H | H | Cl | H |
| H | H | H | H | H | Cl | Cl |
| CN | H | H | H | H | Cl | Cl |
| CN | CN | H | H | H | Cl | Cl |
| CN | H | H | CN | H | Cl | Cl |
| CN | H | H | CN | CN | Cl | Cl |
| CN | H | H | CN | CF₃ | Cl | Cl |
| CF₃ | H | H | H | H | Cl | Cl |
| CF₃ | CF₃ | H | H | H | Cl | Cl |
| CF₃ | H | H | CF₃ | H | Cl | Cl |
| CF₃ | H | H | CF₃ | CF₃ | Cl | Cl |
| CF₃ | H | H | CN | CF₃ | Cl | Cl |
| CN | CF₃ | H | H | H | Cl | Cl |
| CN | H | H | CF₃ | H | Cl | Cl |
| CF₃ | CN | H | H | H | Cl | Cl |
| H | H | H | H | H | H | Br |
| CN | H | H | H | H | H | Br |
| CN | CN | H | H | H | H | Br |
| CN | H | H | CN | H | H | Br |
| CN | H | H | CN | CN | H | Br |
| CN | H | H | CN | CF₃ | H | Br |
| CF₃ | H | H | H | H | H | Br |
| CF₃ | CF₃ | H | H | H | H | Br |
| CF₃ | H | H | CF₃ | H | H | Br |
| CF₃ | H | H | CF₃ | CF₃ | H | Br |
| CF₃ | H | H | CN | CF₃ | H | Br |
| CN | CF₃ | H | H | H | H | Br |
| CN | H | H | CF₃ | H | H | Br |
| CF₃ | CN | H | H | H | H | Br |
| H | H | H | H | H | Br | H |
| CN | H | H | H | H | Br | H |
| CN | CN | H | H | H | Br | H |
| CN | H | H | CN | H | Br | H |
| CN | H | H | CN | CN | Br | H |
| CN | H | H | CN | CF₃ | Br | H |
| CF₃ | H | H | H | H | Br | H |
| CF₃ | CF₃ | H | H | H | Br | H |
| CF₃ | H | H | CF₃ | H | Br | H |
| CF₃ | H | H | CF₃ | CF₃ | Br | H |
| CF₃ | H | H | CN | CF₃ | Br | H |
| CN | CF₃ | H | H | H | Br | H |
| CN | H | H | CF₃ | H | Br | H |
| CF₃ | CN | H | H | H | Br | H |
| H | H | H | H | H | Br | Br |
| CN | H | H | H | H | Br | Br |
| CN | CN | H | H | H | Br | Br |
| CN | H | H | CN | H | Br | Br |
| CN | H | H | CN | CN | Br | Br |
| CN | H | H | CN | CF₃ | Br | Br |
| CF₃ | H | H | H | H | Br | Br |
| CF₃ | CF₃ | H | H | H | Br | Br |
| CF₃ | H | H | CF₃ | H | Br | Br |
| CF₃ | H | H | CF₃ | CF₃ | Br | Br |
| CF₃ | H | H | CN | CF₃ | Br | Br |
| CN | CF₃ | H | H | H | Br | Br |
| CN | H | H | CF₃ | H | Br | Br |
| CF₃ | CN | H | H | H | Br | Br |
| H | H | H | H | H | H | I |
| CN | H | H | H | H | H | I |
| CN | CN | H | H | H | H | I |
| CN | H | H | CN | H | H | I |
| CN | H | H | CN | CN | H | I |
| CN | H | H | CN | CF₃ | H | I |
| CF₃ | H | H | H | H | H | I |
| CF₃ | CF₃ | H | H | H | H | I |
| CF₃ | H | H | CF₃ | H | H | I |
| CF₃ | H | H | CF₃ | CF₃ | H | I |
| CF₃ | H | H | CN | CF₃ | H | I |
| CN | CF₃ | H | H | H | H | I |
| CN | H | H | CF₃ | H | H | I |
| CF₃ | CN | H | H | H | H | I |
| H | H | H | H | H | I | H |
| CN | H | H | H | H | I | H |
| CN | CN | H | H | H | I | H |
| CN | H | H | CN | H | I | H |
| CN | H | H | CN | CN | I | H |
| CN | H | H | CN | CF₃ | I | H |
| CF₃ | H | H | H | H | I | H |
| CF₃ | CF₃ | H | H | H | I | H |
| CF₃ | H | H | CF₃ | H | I | H |
| CF₃ | H | H | CF₃ | CF₃ | I | H |
| CF₃ | H | H | CN | CF₃ | I | H |
| CN | CF₃ | H | H | H | I | H |
| CN | H | H | CF₃ | H | I | H |
| CF₃ | CN | H | H | H | I | H |
| H | H | H | H | H | I | I |
| CN | H | H | H | H | I | I |
| CN | CN | H | H | H | I | I |
| CN | H | H | CN | H | I | I |
| CN | H | H | CN | CN | I | I |
| CN | H | H | CN | CF₃ | I | I |
| CF₃ | H | H | H | H | I | I |
| CF₃ | CF₃ | H | H | H | I | I |
| CF₃ | H | H | CF₃ | H | I | I |
| CF₃ | H | H | CF₃ | CF₃ | I | I |
| CF₃ | H | H | CN | CF₃ | I | I |
| CN | CF₃ | H | H | H | I | I |
| CN | H | H | CF₃ | H | I | I |
| CF₃ | CN | H | H | H | I | I |
| H | H | H | H | H | H | NO₂ |
| CN | H | H | H | H | H | NO₂ |
| CN | CN | H | H | H | H | NO₂ |
| CN | H | H | CN | H | H | NO₂ |
| CN | H | H | CN | CN | H | NO₂ |
| CN | H | H | CN | CF₃ | H | NO₂ |
| CF₃ | H | H | H | H | H | NO₂ |
| CF₃ | CF₃ | H | H | H | H | NO₂ |
| CF₃ | H | H | CF₃ | H | H | NO₂ |
| CF₃ | H | H | CF₃ | CF₃ | H | NO₂ |
| CF₃ | H | H | CN | CF₃ | H | NO₂ |
| CN | CF₃ | H | H | H | H | NO₂ |
| CN | H | H | CF₃ | H | H | NO₂ |
| CF₃ | CN | H | H | H | H | NO₂ |
| H | H | H | H | H | NO₂ | H |
| CN | H | H | H | H | NO₂ | H |
| CN | CN | H | H | H | NO₂ | H |
| CN | H | H | CN | H | NO₂ | H |
| CN | H | H | CN | CN | NO₂ | H |
| CN | H | H | CN | CF₃ | NO₂ | H |
| CF₃ | H | H | H | H | NO₂ | H |
| CF₃ | CF₃ | H | H | H | NO₂ | H |
| CF₃ | H | H | CF₃ | H | NO₂ | H |
| CF₃ | H | H | CF₃ | CF₃ | NO₂ | H |
| CF₃ | H | H | CN | CF₃ | NO₂ | H |
| CN | CF₃ | H | H | H | NO₂ | H |
| CN | H | H | CF₃ | H | NO₂ | H |
| CF₃ | CN | H | H | H | NO₂ | H |
| H | H | H | H | H | NO₂ | NO₂ |
| CN | H | H | H | H | NO₂ | NO₂ |
| CN | CN | H | H | H | NO₂ | NO₂ |
| CN | H | H | CN | H | NO₂ | NO₂ |
| CN | H | H | CN | CN | NO₂ | NO₂ |
| CN | H | H | CN | CF₃ | NO₂ | NO₂ |
| CF₃ | H | H | H | H | NO₂ | NO₂ |
| CF₃ | CF₃ | H | H | H | NO₂ | NO₂ |
| CF₃ | H | H | CF₃ | H | NO₂ | NO₂ |
| CF₃ | H | H | CF₃ | CF₃ | NO₂ | NO₂ |
| CF₃ | H | H | CN | CF₃ | NO₂ | NO₂ |
| CN | CF₃ | H | H | H | NO₂ | NO₂ |
| CN | H | H | CF₃ | H | NO₂ | NO₂ |
| CF₃ | CN | H | H | H | NO₂ | NO₂ |

Hereinafter, specific examples of the compound (I) will be shown. However, the compound (I) is not limited thereto.

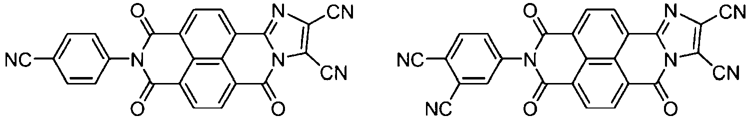

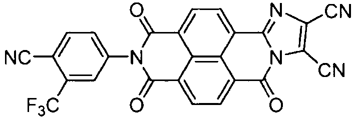

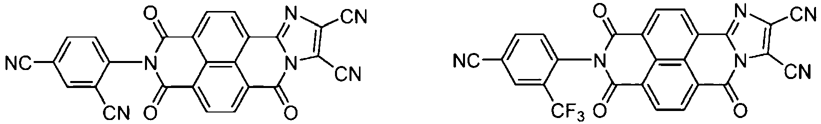

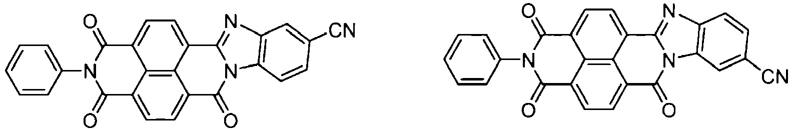

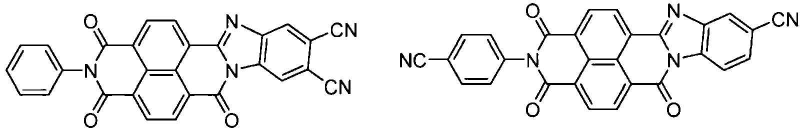

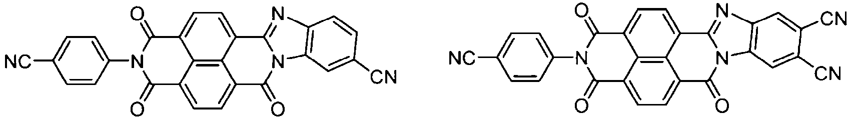

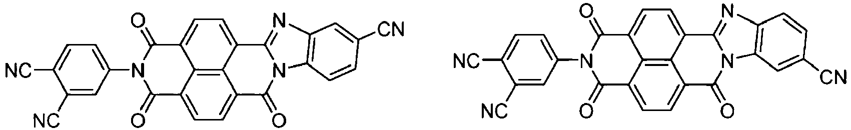

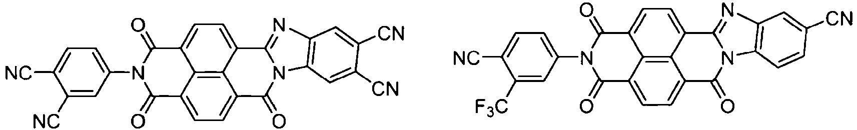

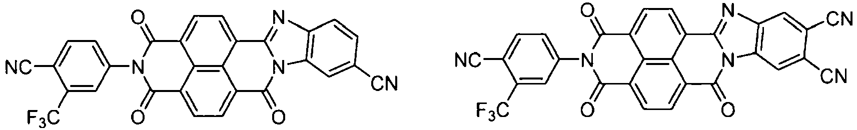

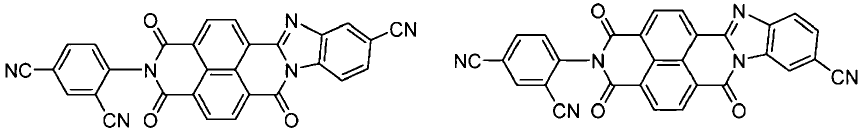

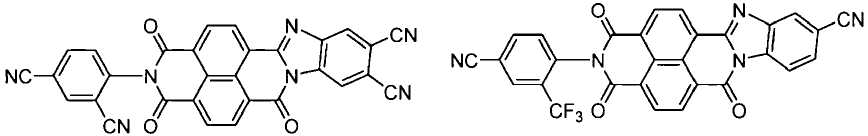

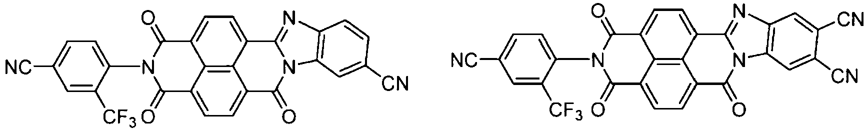

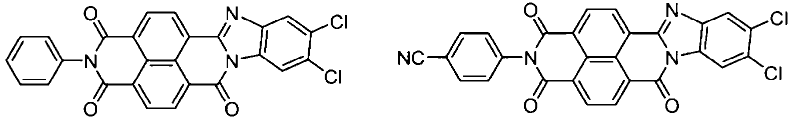

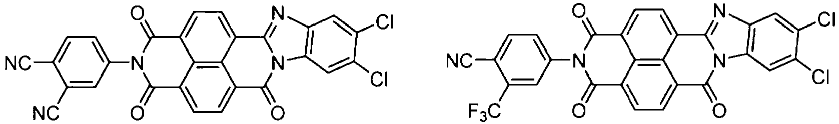

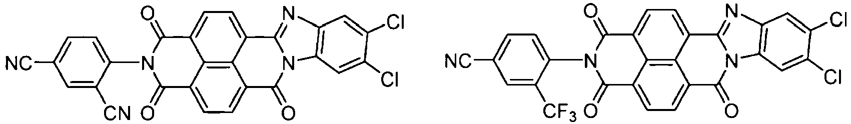

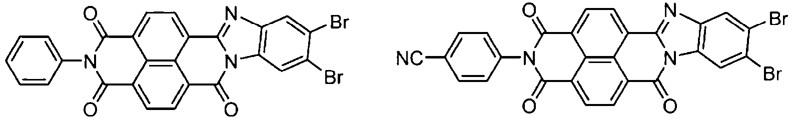

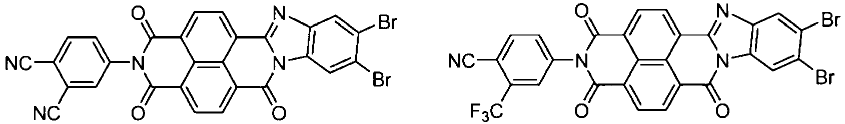

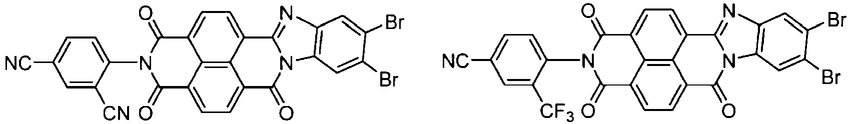

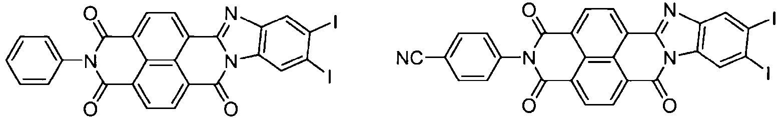

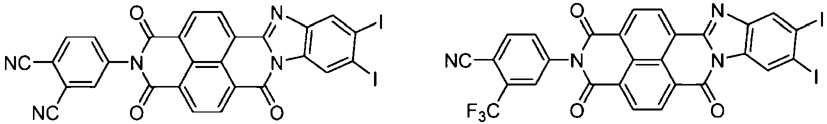

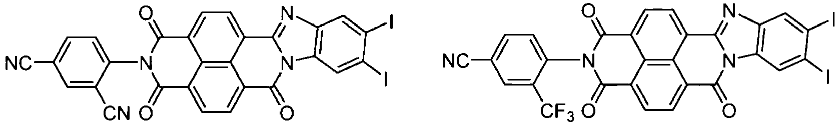

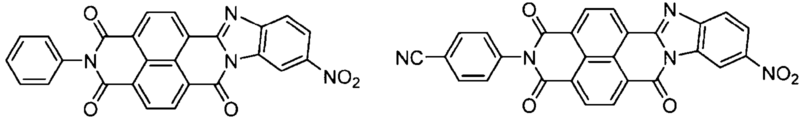

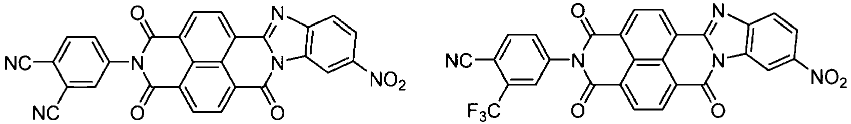

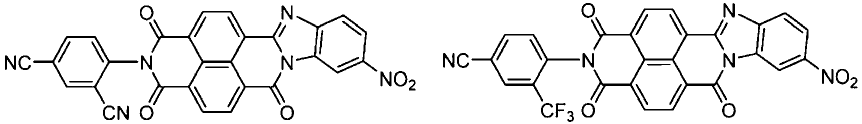

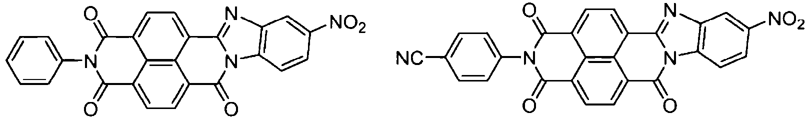

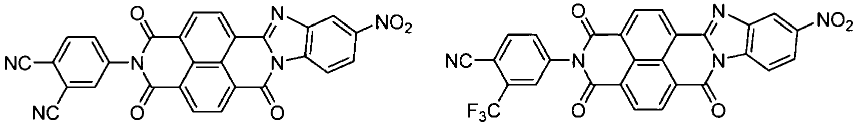

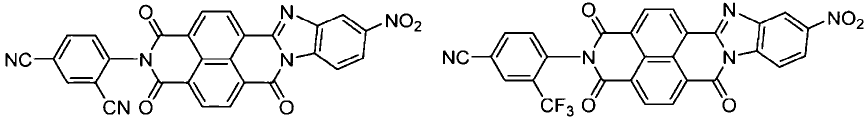

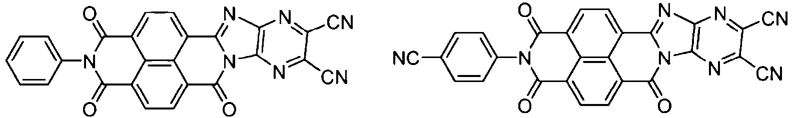

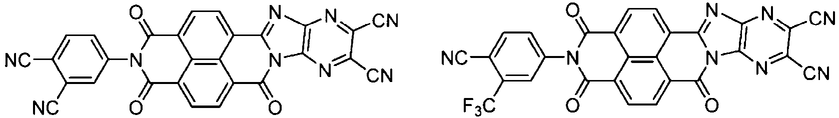

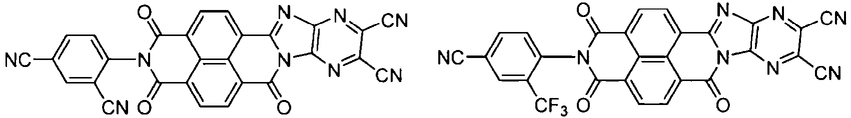

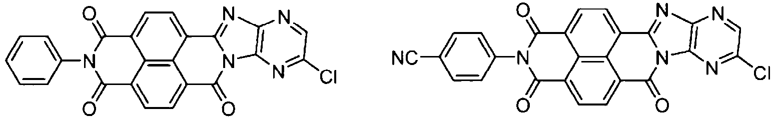

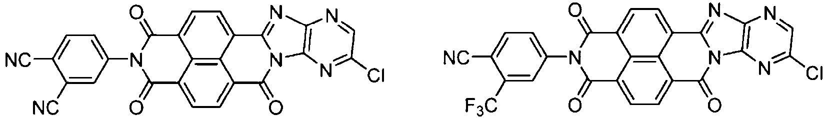

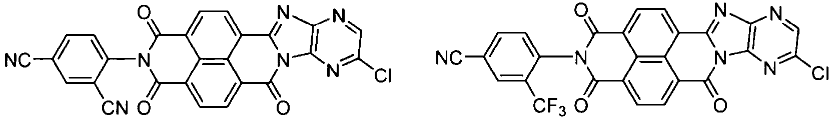

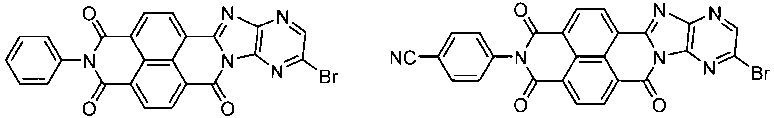

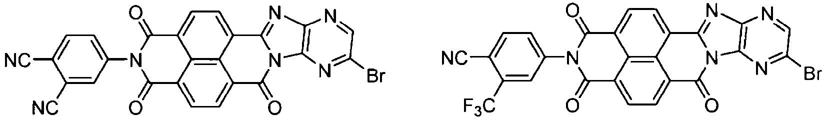

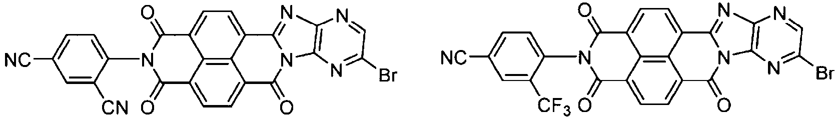

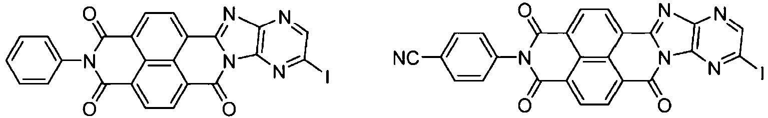

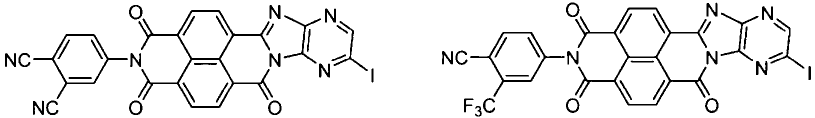

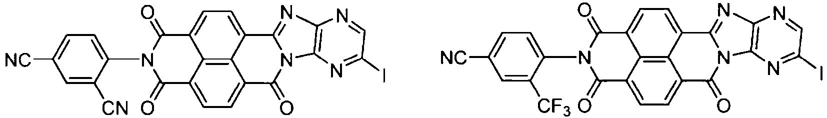

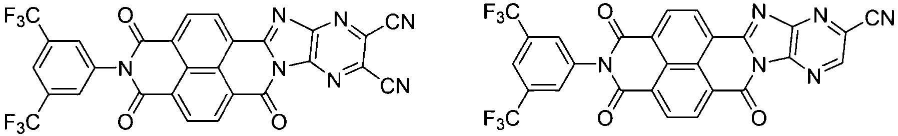

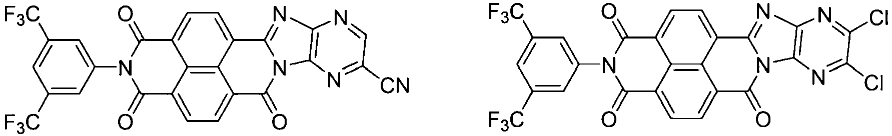

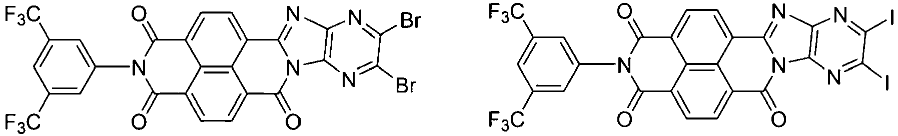

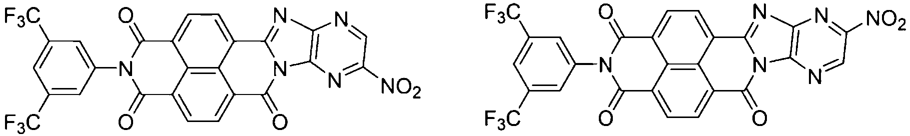

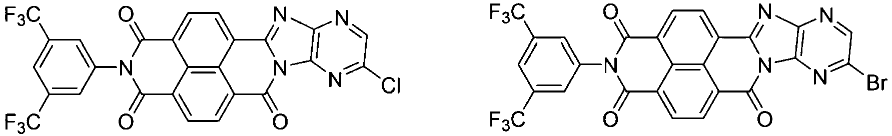

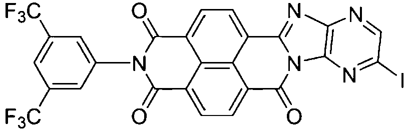

### (Compound (V))

The compound according to another aspect of the present embodiment is represented by the following formula (V) (hereinafter, this compound is also referred to as a "compound (V)"): 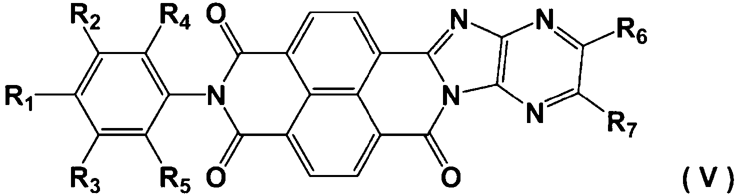 In the formula, R₁, R₂, R₃, R₄, and R₅ are each independently selected from the group consisting of a hydrogen atom, a halogen atom, a hydroxy group, a thiol group, an amino group, a cyano group, a carboxy group, a nitro group, and an optionally substituted linear, branched or cyclic alkyl group, thioalkyl group, thioaryl group, arylsulfonyl group, aryloxy group, alkylsulfonyl group, alkylamino group, arylamino group, alkoxy group, acylamino group, acyloxy group, aryl group, carboxyamide group, carboalkoxy group, carboaryloxy group, acyl group, and monovalent heterocyclic group, and one or more of R₁, R₂, R₃, R₄, and R₅ is a halogen atom or a monovalent organic group having a Hammett substituent constant σp of 0.10 or more. R₆ and R₇ are each independently a hydrogen atom, a halogen atom, or a monovalent organic group, one or more of R₆ and R₇ is a halogen atom or a monovalent organic group having a Hammett substituent constant σp of 0.10 or more, any adjacent members among R₁, R₂, R₃, R₄, R₅, R₆, and R₇ optionally constitute a portion of a condensed aliphatic ring or a condensed aromatic ring, and the condensed aliphatic ring and the condensed aromatic ring each optionally contain one or more atoms other than a carbon atom.

From the viewpoint of more effectively and reliably exerting the working effects according to the present invention, R₆ and R₇ are preferably each independently selected from the group consisting of a hydrogen atom, a fluorine atom, a hydroxy group, a thiol group, an amino group, a cyano group, a carboxy group, a nitro group, and an optionally substituted linear, branched or cyclic alkyl group, thioalkyl group, thioaryl group, arylsulfonyl group, aryloxy group, alkylsulfonyl group, alkylamino group, arylamino group, alkoxy group, acylamino group, acyloxy group, aryl group, carboxyamide group, carboalkoxy group, carboaryloxy group, acyl group, and monovalent heterocyclic group, any adjacent members among R₁, R₂, R₃, R₄, R₅, R₆, and R₇ optionally constitute a portion of a condensed aliphatic ring or a condensed aromatic ring, and the condensed aliphatic ring and the condensed aromatic ring each optionally contain one or more atoms other than a carbon atom.

Such a compound (V) can suppress leak current in the dark and has excellent wavelength selectivity, and exhibits excellent characteristics, particularly, as a photoelectric conversion element material. A factor for this is not certain and is considered as follows by the present inventors, though the factor is not limited thereto: that is, when R₁, R₂, R₃, R₄, R₅, R₆, and R₇ have the above-described Hammett substituent constants σp and a structure with an extended π-conjugated system, the energy level of the lowest unoccupied molecular orbital of the compound (V) is lowered, enabling suppression of leak current in the dark and providing excellent wavelength selectivity.

In the compound (V) of the present embodiment, without particular limitations, R₁, R₂, R₃, R₄, and R₅ are more preferably each independently selected from the group consisting of a hydrogen atom, a halogen atom, a nitro group, a cyano group, and an optionally substituted linear, branched or cyclic alkyl group, and particularly preferably each independently selected from the group consisting of a hydrogen atom, a halogen atom, a nitro group, a cyano group, and a halogen atom-substituted alkyl group. The compound (V) having the structure mentioned above can further suppress leak current in the dark and tends to have better wavelength selectivity.

In the compound (V) of the present embodiment, R₁, R₂, R₃, R₄, and R₅ may be the same or different. Without particular limitations, at least two moieties selected from R₁, R₂, R₃, R₄, and R₅ are preferably the same, and at least three moieties selected from R₁, R₂, R₃, R₄, and R₅ are more preferably the same, from the viewpoint of more effectively and reliably exerting the working effects according to the present invention.

In the compound (V) of the present embodiment, from the viewpoint of more effectively and reliably exerting the working effects according to the present invention, without particular limitations, at least one of R₁, R₂, R₃, R₄, and R₅ is preferably not a hydrogen atom; at least one of R₁, R₂, R₃, R₄, and R₅ is more preferably selected from the group consisting of a halogen atom, a nitro group, a cyano group, and an optionally substituted linear, branched or cyclic alkyl group; and at least one of R₁, R₂, R₃, R₄, and R₅ is particularly preferably selected from the group consisting of a halogen atom, a nitro group, a cyano group, and a halogen atom-substituted alkyl group. Moreover, at least R₁ is preferably selected from the group consisting of a halogen atom, a nitro group, a cyano group, and a halogen atom-substituted alkyl group, and is more preferably a cyano group.

In the compound (V) of the present embodiment, without particular limitations, R₆ and R₇ are preferably each independently selected from the group consisting of a hydrogen atom, a fluorine atom, a hydroxy group, a thiol group, an amino group, a cyano group, a carboxy group, a nitro group, and an optionally substituted linear, branched or cyclic alkyl group, thioalkyl group, thioaryl group, arylsulfonyl group, aryloxy group, alkylsulfonyl group, alkylamino group, arylamino group, alkoxy group, acylamino group, acyloxy group, aryl group, carboxyamide group, carboalkoxy group, carboaryloxy group, acyl group, and monovalent heterocyclic group, more preferably each independently selected from the group consisting of a hydrogen atom, a fluorine atom, a nitro group, a cyano group, and an optionally substituted linear, branched or cyclic alkyl group, and particularly preferably each independently selected from the group consisting of a hydrogen atom, a fluorine atom, a nitro group, a cyano group, and a halogen atom-substituted alkyl group. The compound (V) having the structure mentioned above can further suppress leak current in the dark and tends to have better wavelength selectivity.

In the compound (V) of the present embodiment, preferred Hammett substituent constants σp of R₁, R₂, R₃, R₄, R₅, R₆, and R₇ may be the same as the Hammett substituent constants σp in the compound (I).

In the compound (V) of the present embodiment, R₆ and R₇ may be the same or different.

Hereinafter, preferred combinations of R₁, R₂, R₃, R₄, R₅, R₆, and R₇ in the compound (V) will be shown.

| R₁ | R₂ | R₃ | R₄ | R₅ | R₆ | R₇ |
|---|---|---|---|---|---|---|
| H | H | H | H | H | H | F |
| CN | H | H | H | H | H | F |
| CN | CN | H | H | H | H | F |
| CN | H | H | CN | H | H | F |
| CN | H | H | CN | CN | H | F |
| CN | H | H | CN | CF₃ | H | F |
| CF₃ | H | H | H | H | H | F |
| CF₃ | CF₃ | H | H | H | H | F |
| CF₃ | H | H | CF₃ | H | H | F |
| CF₃ | H | H | CF₃ | CF₃ | H | F |
| CF₃ | H | H | CN | CF₃ | H | F |
| CN | CF₃ | H | H | H | H | F |
| CN | H | H | CF₃ | H | H | F |
| CF₃ | CN | H | H | H | H | F |
| H | H | H | H | H | F | H |
| CN | H | H | H | H | F | H |
| CN | CN | H | H | H | F | H |
| CN | H | H | CN | H | F | H |
| CN | H | H | CN | CN | F | H |
| CN | H | H | CN | CF₃ | F | H |
| CF₃ | H | H | H | H | F | H |
| CF₃ | CF₃ | H | H | H | F | H |
| CF₃ | H | H | CF₃ | H | F | H |
| CF₃ | H | H | CF₃ | CF₃ | F | H |
| CF₃ | H | H | CN | CF₃ | F | H |
| CN | CF₃ | H | H | H | F | H |
| CN | H | H | CF₃ | H | F | H |
| CF₃ | CN | H | H | H | F | H |
| H | H | H | H | H | F | F |
| CN | H | H | H | H | F | F |
| CN | CN | H | H | H | F | F |
| CN | H | H | CN | H | F | F |
| CN | H | H | CN | CN | F | F |
| CN | H | H | CN | CF₃ | F | F |
| CF₃ | H | H | H | H | F | F |
| CF₃ | CF₃ | H | H | H | F | F |
| CF₃ | H | H | CF₃ | H | F | F |
| CF₃ | H | H | CF₃ | CF₃ | F | F |
| CF₃ | H | H | CN | CF₃ | F | F |
| CN | CF₃ | H | H | H | F | F |
| CN | H | H | CF₃ | H | F | F |
| CF₃ | CN | H | H | H | F | F |

Hereinafter, specific examples of the compound (V) will be shown. However, the compound (V) is not limited thereto.

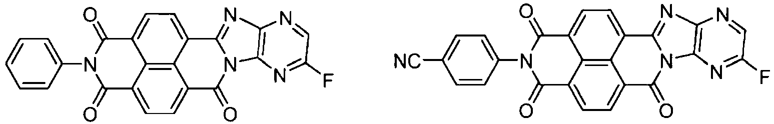

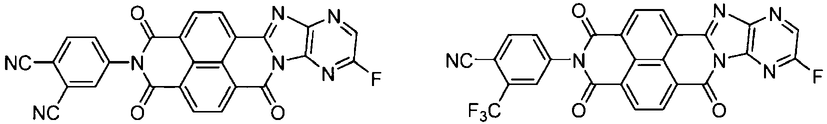

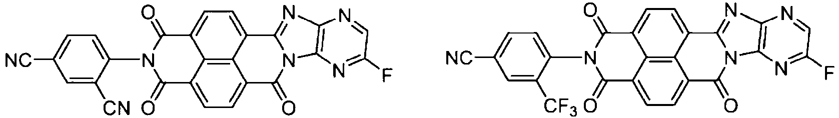

### (Compound (VI))

The compound according to another aspect that may be used in the image sensor of the present embodiment described later is represented by the following formula (VI) (hereinafter, this compound is also referred to as a "compound (VI)"): 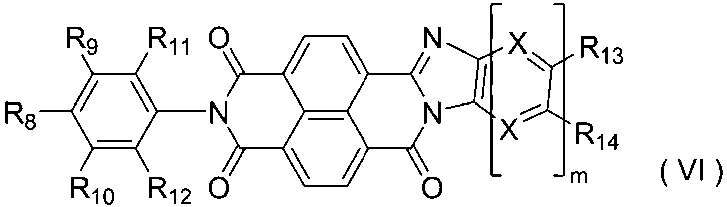 In the formula, X is each independently selected from the group consisting of a methine group and a nitrogen atom, and m is an integer of 0 or more and 3 or less. R₈, R₉, R₁₀, R₁₁, and R₁₂ are each independently a hydrogen atom, a halogen atom having a Hammett substituent constant σp of less than 0.10, or a monovalent organic group having a Hammett substituent constant σp of less than 0.10. R₁₃ and R₁₄ are each independently a hydrogen atom, a halogen atom, or a monovalent organic group, and one or more of R₁₃ and R₁₄ is a halogen atom or a monovalent organic group having a Hammett substituent constant σp of 0.10 or more. Any adjacent members among R₈, R₉, R₁₀, R₁₁, R₁₂, R₁₃, and R₁₄ optionally constitute a portion of a condensed aliphatic ring or a condensed aromatic ring, and the condensed aliphatic ring and the condensed aromatic ring each optionally contain one or more atoms other than a carbon atom.

Such a compound (VI) can suppress leak current in the dark and has excellent wavelength selectivity, and is therefore useful particularly when used in an image sensor. A factor for this is not certain and is considered as follows by the present inventors, though the factor is not limited thereto: that is, when having a structure with an extended π-conjugated system, the energy level of the lowest unoccupied molecular orbital of the compound (V) is lowered, enabling suppression of leak current in the dark and providing excellent wavelength selectivity.

The compound (VI) may be the same as the compound (I), except that R₈, R₉, R₁₀, R₁₁, and R₁₂ are each independently a hydrogen atom, a halogen atom having a Hammett substituent constant σp of less than 0.10, or a monovalent organic group having a Hammett substituent constant σp of less than 0.10, and that R₁₃ and R₁₄ may be fluorine atoms. That is, R₁₃ and R₁₄ in the formula (VI) correspond to R₆ and R₇ in the formula (1).

Examples of the monovalent group having a Hammett substituent constant σp of less than 0.1 include a fluorine atom, a methyl group, a methoxy group, an amino group, and a hydroxy group.

The energy level of the lowest unoccupied molecular orbital (LUMO) obtained by density functional formalism of the compound (I), compound (V), and compound (VI) of the present embodiment (hereinafter, simply referred to as the "compounds (I), (V), and (VI)") is preferably -6.00 eV or more and -3.60 eV or less, more preferably -5.50 eV or more and -3.65 eV or less, from the viewpoint of more effectively and reliably exerting the working effects according to the present invention. The compounds (I), (V), and (VI) of the present embodiment can be structurally optimized by molecular simulation using density functional formalism (e.g., molecular simulation using quantum chemical calculation program Gaussian manufactured by Gaussian, Inc.) to determine the energy level of the lowest unoccupied molecular orbital. The energy level of the lowest unoccupied molecular orbital obtained by density functional formalism of the compounds (I), (V), and (VI) of the present embodiment may be adjusted, without particular limitations, by changing R₁ to R₇ and R₈ to R₁₄. From the viewpoint of setting the energy level of the lowest unoccupied molecular orbital of each of the above compounds to within the range described above, in the compounds (I), (V), and (VI), without particular limitations, at least one of R₁, R₂, R₃, R₄, R₅, R₆, and R₇, or of R₈, R₉, R₁₀, R₁₁, R₁₂, R₁₃, and R₁₄, is preferably an electron-withdrawing group, and at least one of R₁, R₂, R₃, R₄, R₅, R₆, and R₇, or of R₈, R₉, R₁₀, R₁₁, R₁₂, R₁₃, and R₁₄, is more preferably a cyano group or a halogen atom-substituted alkyl group.

The difference (eV) between the energy level of the lowest unoccupied molecular orbital and the energy level of the highest occupied molecular orbital (HOMO) ([energy level of the highest occupied molecular orbital] - [energy level of the lowest unoccupied molecular orbital]) obtained by density functional formalism of the compounds (I), (V), and (VI) of the present embodiment is preferably 2,85 eV or more and 4.00 eV or less. The compounds (I), (V) and (VI) having the difference in energy level that falls within the range described above tends to be able to reduce leak current in the dark when used as a photoelectric conversion element material.

The molecular weight of the compounds (I), (V) and (VI) of the present embodiment is preferably 350 or higher, more preferably 370 or higher, further preferably 390 or higher. When the molecular weight is 350 or higher, change in physical properties caused by the thermal motion of molecules that may occur in heating operation in an organic thin film production process or a high-temperature environment using the compounds (I), (V) and (VI) can be more suppressed. Particularly, in the case of forming the compounds (I), (V) and (VI) by vacuum vapor deposition, the molecular weight of the compounds (I), (V) and (VI) is preferably 1000 or lower, more preferably 950 or lower, further preferably 900 or lower. When the molecular weight is 1000 or lower, heat energy necessary for sublimation in forming an organic thin film of the compounds (I), (V) and (VI) by vacuum vapor deposition can be kept lower. A favorable thin film can thereby be formed without thermally deteriorating the compounds (I), (V) and (VI). However, in the case of forming a thin film by solution coating, the molecular weight of the compounds (I), (V) and (VI) may be larger than 1000 because such a problem is unlikely to occur.

In the compounds (I), (V), and (VI) of the present embodiment, it is preferable that the temperature at which the mass is reduced by 5% from the mass before heating due to heating, as measured under the following Condition 1 (hereinafter, also referred to as "5% mass reduction temperature"), is 430°C or higher and 550°C or lower. When the 5% mass reduction temperature is within the range described above, change in physical properties caused by the thermal motion of molecules that may occur in heating operation in an organic thin film production process or a high-temperature environment using the compounds (I), (V), and (VI) can be more suppressed.
(Condition 1)
Pressure: 101325 Pa
Nitrogen: 200 mL/min
Temperature increase condition: increased from 20°C to 550°C at 10°C/min.

In the compounds (I), (V), and (VI) of the present embodiment, it is preferable that the temperature at which the mass is reduced by 10% from the mass before heating due to heating, as measured under the following Condition 2 (hereinafter, also referred to as "10% mass reduction temperature"), is 220°C or higher and 400°C or lower. When the 10% mass reduction temperature is within the range described above, change in physical properties caused by the thermal motion of molecules that may occur in heating operation in an organic thin film production process or a high-temperature environment using the compounds (I), (V), and (VI) can be more suppressed.
(Condition 2)
Pressure: 1 Pa
Temperature increase condition: increased from 20°C to 450°C at 10°C/min.

The compounds (I), (V), and (VI) can be synthesized by, for example, the following scheme. Note that, in the compound (VI), R₁, R₂, R₃, R₄, R₅, R₆, and R₇ in the following scheme may be replaced with R₈, R₉, R₁₀, R₁₁, R₁₂, R₁₃, and R₁₄, respectively.

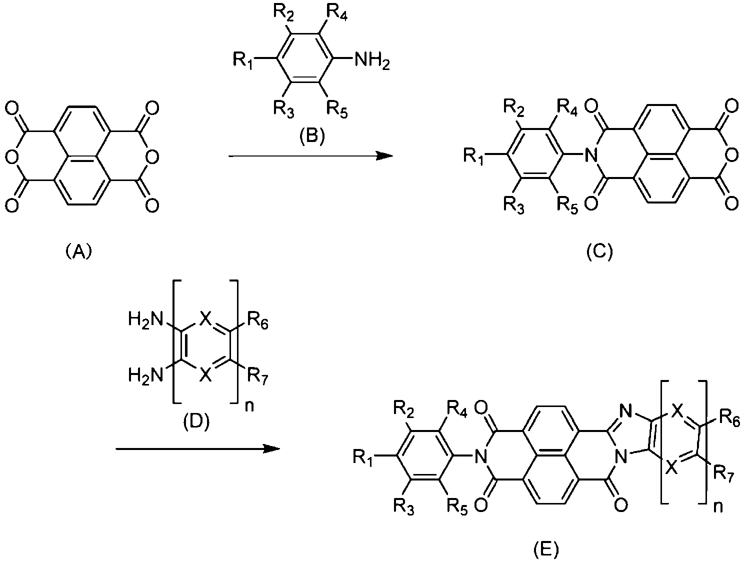

More specifically, for example, by an imide reaction from commercially available (A) using a compound (B), a compound (C) can be obtained. Moreover, by further carrying out an addition reaction of a compound (D), a compound (E) can be obtained. More specifically, the synthesis can be performed with reference to the method described in Japanese Translation of PCT International Application Publication No. 2014-520394. The imide reaction and addition reaction may be carried out using a compound in which R₁ to R₇ are introduced, or R₁ to R₇ may be introduced after carrying out the imide reaction and addition reaction.

### (Compound (II), Compound (III), and Compound (IV))

In the present embodiment, compounds represented by the following formula (II), formula (III), and formula (IV) (hereinafter, these compounds are also referred to as the "compound (II), compound (III), and compound (IV)") can be synthesized by the methods described in Japanese Translation of PCT International Application Publication No. 2014-520394 and Japanese Translation of PCT International Application Publication No. 2020-524404.

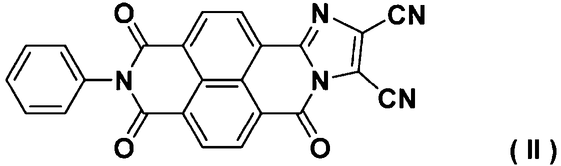

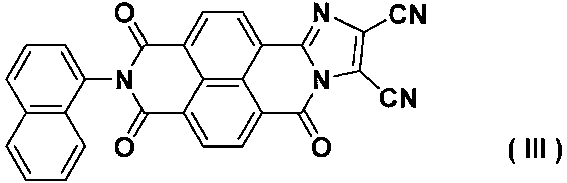

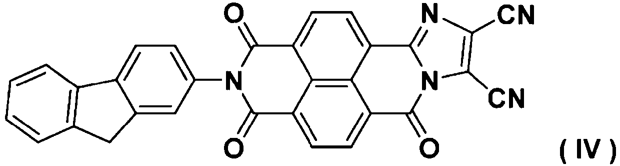

The compound (I), compound (II), compound (III), compound (IV), compound (V), and compound (VI) of the present embodiment (hereinafter, simply referred to as the "compounds (I) to (VI)") can be obtained by, for example, synthesis as described above. The content ratio of the compounds (I) to (VI) in the product obtained by synthesis (100% by mass) is preferably 90% by mass or more, more preferably 93% by mass, and further preferably 97% by mass or more. When the content ratio of the above compounds is 90% by mass or more, the trap of a carrier to an impurity level resulting from unintended impurities upon use of the above compounds in a photoelectric conversion element or an image sensor can be more effectively and reliably avoided. As a result, a photoelectric conversion element or an image sensor having better performance can be obtained by suppressing carrier recombination. The content ratio can be measured by liquid chromatography, gas chromatography, elemental analysis, or the like, and a method known in the art can be used.

### (Material for photoelectric conversion element)

The compounds (I) to (VI) of the present embodiment are used as a photoelectric conversion element material. More specifically, the compounds (I) to (VI) are used as a material contained in each layer in a photoelectric conversion element mentioned later. Among such layers, the compounds (I) to (VI) are preferably contained in a photoelectric conversion film, more preferably contained in an auxiliary layer, and particularly preferably contained in at least one of an electron transport layer and a hole blocking layer, from the viewpoint of more effectively and reliably exerting the working effects according to the present invention.

The compounds (I) to (VI) of the present embodiment can be directly used as a photosensitive material or may be mixed with an additional material and used as a photosensitive composition. The content of the compounds (I) to (VI) in the photosensitive composition may be 50% by mass or more based on the total amount of the composition. The content may be 95% by mass or less, may be 90% by mass or less, or may be 80% by mass or less. The material other than the compounds (I) to (VI) in the photosensitive composition is not particularly limited as long as the material can be contained in a usual photosensitive composition. Examples of such a material include n-type semiconductor materials, p-type semiconductor materials, and light-absorbing materials mentioned later. These materials can each be used singly or in combination of two or more thereof.

### (Organic thin film)

The organic thin film of the present embodiment contains the compounds (I) to (VI) of the present embodiment or the material for a photoelectric conversion element. Such an organic thin film can be prepared by a general dry film formation method or wet film formation method. Specific examples thereof include: resistance heating vapor deposition, electron beam vapor deposition, sputtering, and molecular lamination methods which are vacuum processes; casting which is a solution process; coating methods such as spin coating, dip coating, blade coating, wire bar coating, and spray coating; printing methods such as inkjet printing, screen printing, offset printing, and relief printing; and soft lithography approaches such as microcontact printing methods. It is generally desirable to be able to use the material for a photoelectric conversion element in a process of coating with the compound in a solution state, from the viewpoint of the easy processing. However, for a photoelectric conversion element as prepared by laminating organic thin films, a dry film formation method such as resistance heating vapor deposition is preferred because a coating solution might infiltrate into a film of a lower layer.

For example, in the dry film formation method, the material for a photoelectric conversion element of the present embodiment, and optionally, an additional material appropriate for the application of the photoelectric conversion element are mixed to prepare a composition, and the composition can then be vapor-deposited onto a base material or another film in vacuum to obtain an organic thin film. In the wet film formation method, the photoelectric conversion film of the present embodiment, and optionally, an additional material for the application of the photoelectric conversion element are mixed together with a solvent to prepare a liquid composition, with which a base material or another film can then be coated and printed, followed by drying to obtain an organic thin film.

The organic thin film of the present embodiment may contain a material other than the compounds (I) to (VI) serving as the material for a photoelectric conversion element of the present embodiment. The content of the compounds (I) to (VI) in the organic thin film of the present embodiment is not particularly limited as long as performance necessary for use as the material for a photoelectric conversion element is exerted. The content of the compounds (I) to (VI) may be, for example, 50% by mass or more based on the total amount of the organic thin film. The content is preferably 80% by mass or more, more preferably 90% by mass or more, further preferably 95% by mass or more, from the viewpoint of more effectively and reliably exerting the working effects according to the present invention. The upper limit of the content of the compounds (I) to (VI) may be 100% by mass. When the organic thin film of the present embodiment contains a material other than the compounds (I) to (VI), the material is not particularly limited as long as the material can be used as a usual material for a photoelectric conversion element. Examples of such a material include n-type semiconductor materials, p-type semiconductor materials, and light-absorbing materials mentioned later, molybdenum oxide called doping material, alkali metals, and alkali metal compounds. These materials can each be used singly or in combination of two or more thereof.

The thickness of the organic thin film depends on the resistance value and/or charge mobility of each substance and thus cannot be limited. The thickness is usually 0.5 nm or more and 5000 nm or less and may be 1 nm or more and 1000 nm or less or may be 5 nm or more and 500 nm or less.

The organic thin film of the present embodiment preferably has a local maximum absorption wavelength of an optical absorption band at 450 nm or less from the viewpoint of more effectively and reliably exerting the working effects according to the present invention.

### (Photoelectric conversion element)

The photoelectric conversion element of the present embodiment refers to an element that generates a charge in response to the quantity of an incident light, and outputs the generated charge to the outside of the photoelectric conversion element through a condenser (hereinafter, also referred to as an "accumulation unit") for charge accumulation, a transistor circuit (hereinafter, also referred to as a "readout unit") for readout, and the like. In this context, the photoelectric conversion element refers to an element having a photoelectric conversion film, which absorbs at least a portion of incident lights, disposed between a pair of opposed electrodes, and a light is incident on the photoelectric conversion element from above the electrodes. The photoelectric conversion film is a photosensitive thin film comprising a material that absorbs at least a portion of incident lights in the infrared region, and generates holes and electrons as a result of light incidence. The photoelectric conversion element of the present embodiment may have a photoelectric conversion element which generates a charge in response to the quantity of an incident light in the infrared region (hereinafter, also referred to as an "infrared photoelectric conversion element"). In this context, the infrared photoelectric conversion element refers to an element having a photoelectric conversion film, which absorbs infrared light (hereinafter, also referred to as "infrared photoelectric conversion film"), disposed between a pair of opposed electrodes, and a light is incident on the infrared photoelectric conversion element from above the electrodes. The infrared photoelectric conversion film is a photosensitive thin film comprising a material that absorbs at least a portion of incident lights in the infrared region (hereinafter, referred to as an "infrared absorptive material"), and generates holes and electrons as a result of light incidence.

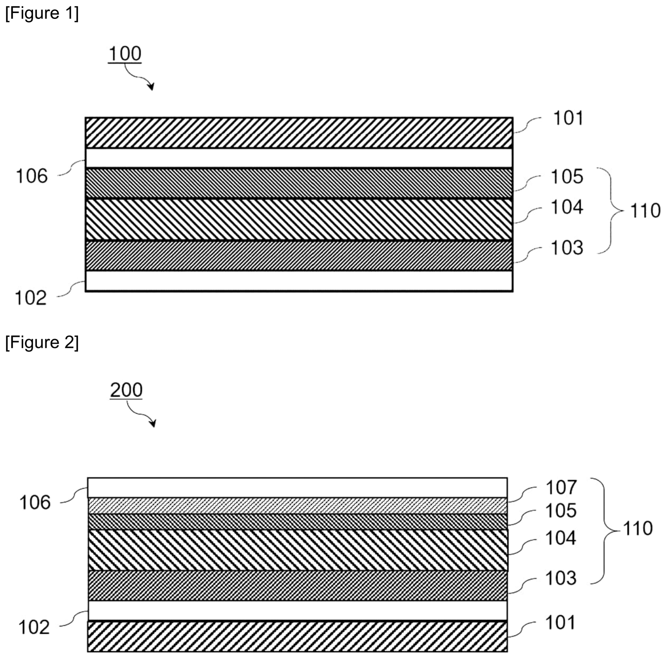

The photoelectric conversion element of the present embodiment will be described with appropriate reference to Figure 1. A photoelectric conversion element 100 has a lower electrode 102 which is a first electrode film, an upper electrode 106 which is a second electrode film, and a photoelectric conversion film 110 positioned between the lower electrode 102 and the upper electrode 106. The photoelectric conversion element 100 may have a base material 101 which usually has insulation properties on the side, opposite to the photoelectric conversion film 110, of the upper electrode 106.

When the photoelectric conversion film 110 has hole-transporting properties or electron-transporting properties, the lower electrode 102 and the upper electrode 106 plays a role in extracting holes from the photoelectric conversion film 110 and collecting the holes, or extracting electrons from the photoelectric conversion film 110 and discharging the electrons. The material that may be used in these electrodes is not particularly limited as long as it has conductivity to some extent. The material is preferably selected in consideration of close contact with the adjacent photoelectric conversion film 110, electron affinity, an ionization potential and stability, etc. Examples of the material that may be used in the electrode include: conductive metal oxides such as tin oxide (NESA), indium oxide, indium tin oxide (ITO) and indium zinc oxide (IZO); metals such as gold, silver, platinum, chromium, aluminum, iron, cobalt, nickel and tungsten: inorganic conductive substances such as copper iodide and copper sulfide; conductive polymers such as polythiophene, polypyrrole and polyaniline; and carbon. These materials may each be used singly, may be used as a mixture of two or more thereof.

The lower electrode 102 which is the first electrode film is made of a conductive film having light permeability and made of, for example, indium tin oxide (ITO). The material constituting the lower electrode 102 is not limited to ITO. Examples thereof include tin oxide (SnO₂) materials supplemented with a dopant, and zinc oxide materials obtained by adding a dopant to zinc oxide (ZnO). Examples of the zinc oxide material include aluminum zinc oxide (AZO) obtained by adding aluminum (Al) as a dopant, gallium zinc oxide (GZO) obtained by adding gallium (Ga) thereas, and indium zinc oxide (IZO) obtained by adding indium (In) thereas. Alternative examples of the material constituting the lower electrode 102 also include Cul, InSbO₄, ZnMgO, CuInO₂, MgIN₂O₄, CdO, and ZnSnO₃. The thickness of the lower electrode 102 is, for example, 5 nm or more and 3000 nm or less and may be 5 nm or more and 500 nm or less or may be 10 nm or more and 300 nm or less.

The upper electrode 106 which is the second electrode film may be constituted by the same conductive film having light permeability as that of the lower electrode 102, or may be constituted by a metal, such as aluminum, which is usually used in an electrode in the photoelectric conversion element. In a solid-state imaging apparatus using a solid image sensor as one pixel, this upper electrode 106 may be isolated on a pixel basis or may be formed as a common electrode among the respective pixels. The thickness of the upper electrode 106 is, for example, 5 nm or more and 3000 nm or less and may be 5 nm or more and 500 nm or less or may be 10 nm or more and 300 nm or less.

The conductivity of the material for use in the electrodes such as the first electrode film and the second electrode film is not particularly limited as long as it does not hinder the light reception of the photoelectric conversion element more than necessary. The conductivity is preferably as high as possible from the viewpoint of the signal intensity and power consumption of the photoelectric conversion element. For example, as for a transparent electrode, an ITO film having conductivity equal to or less than a sheet resistance value of 300 ohms per square sufficiently functions as the electrode. However, a commercially available product of a substrate having an ITO film having conductivity on the order of several ohms per square (e.g., 5 to 9 ohms per square) is also obtainable, and such a substrate having high conductivity is desirable.

In the case of using an ITO film, the thickness of the electrode can be arbitrarily selected in consideration of conductivity and is usually 5 nm or more and 3000 nm or less, preferably 10 nm or more and 300 nm or less. Examples of the method for forming the film such as ITO include vapor deposition methods, electron beam methods, sputtering methods, chemical reaction methods and coating methods heretofore known in the art. The ITO film disposed on the substrate may be subjected, if necessary, to UV-ozone treatment or plasma treatment.

In the case of laminating a plurality of photoelectric conversion films differing in wavelength to be detected, the film of an electrode for use between the respective photoelectric conversion films needs to be transmissive to a light having a wavelength other than the lights to be detected by the respective photoelectric conversion films. From such a viewpoint, a material transmissive to 90% more of an incident light is preferably used in the film of the electrode, and a material transmissive to 95% or more of a light is more preferably used. The film of the electrode is the film of an electrode other than the pair of electrodes described above.

In the case of further establishing a visible photoelectric conversion unit that senses an infrared light or lights in different visible light regions beneath the photoelectric conversion element according to the present embodiment, the electrode for use in the photoelectric conversion element preferably has a transmittance of 90% or more, more preferably 95% or more, to the visible lights and the infrared light.

The material for the electrode that satisfies such conditions is preferably transparent conducting oxide (TCO) having a high transmittance to visible light and infrared light and a small resistance value. Although a thin film of a metal such as gold can be used as an electrode, its resistance value is extremely increased if the transmittance is adjusted to 90% or more. Thus, TCO is preferred for the electrode. The TCO is particularly preferably ITO, IZO, AZO, FTO, SnO₂, TiO₂ or ZnO₂.

The method for forming the electrode is not particularly limited and can be appropriately selected in consideration of aptitude for the electrode material. In the case of using a transparent electrode, specific examples of the formation method therefor include wet schemes such as printing schemes and coating schemes, physical schemes such as vacuum vapor deposition methods, sputtering methods and ion plating methods, and chemical schemes such as CVD and plasma CVD. When the electrode material is transparent conducting metal oxide such as ITO, examples of the formation method therefor include electron beam methods, sputtering methods, resistance heating vapor deposition methods, chemical reaction methods (e.g., a sol-gel method), and methods of coating with a dispersion of the metal oxide. The film of transparent conducting metal oxide such as ITO may be further subjected to UV-ozone treatment and plasma treatment.

Furthermore, from the viewpoint of more effectively and reliably exerting the working effects according to the present invention, the photoelectric conversion element of the present embodiment is preferably a photoelectric conversion element comprising a first electrode film, a second electrode film, and a photoelectric conversion film positioned between the first electrode film and the second electrode film, wherein the photoelectric conversion film comprises a photoelectric conversion layer and two auxiliary layers positioned between the photoelectric conversion layer and the second electrode film, wherein among the two auxiliary layers, the auxiliary layer closer to the second electrode film comprises the compounds (I) to (VI) of the present embodiment.

Although the factor by which such a photoelectric conversion element can suppress leak current in the dark is not certain, the present inventors consider the following. The photoelectric conversion element of the present embodiment comprises two auxiliary layers between the photoelectric conversion layer and the second electrode film, wherein the auxiliary layer closer to the second electrode film comprises the compounds (I) to (VI). As a result, a rectifying effect is obtained in which the relatively low HOMO levels possessed by the compounds (I) to (VI) suppress the movement of electrons generated in the photoelectric conversion layer toward the second electrode film, and accordingly, it is considered possible to suppress leak current in the dark (hereinafter, also referred to as "dark current"). However, the factor is not limited thereto. Moreover, the photoelectric conversion element of the present embodiment can also exhibit high photoelectric conversion efficiency. This is considered to be because the auxiliary layer closer to the second electrode film comprises the compounds (I) to (VI), which enhances the chemical affinity between the second electrode film and the photoelectric conversion film, and can make the energy gradient for moving electrons to the second electrode film smoother. However, the factor is not limited thereto.

As one aspect of the present embodiment, the photoelectric conversion film 110 may comprise the material for a photoelectric conversion element of the present embodiment or may include the organic thin film described above. More specifically, the photoelectric conversion film 110 has, for example, a photoelectric conversion layer 104, a first auxiliary layer 103 positioned on the lower electrode film 102 side of the photoelectric conversion layer 104, and a second auxiliary layer 105 positioned on the upper electrode film 106 side of the photoelectric conversion layer 104. The photoelectric conversion film 110 shown in Figure 1 has the first auxiliary layer 103 and the second auxiliary layer 105, while the photoelectric conversion film may have only one of these auxiliary layers. Alternatively, the photoelectric conversion film may have neither of these auxiliary layers and have only the photoelectric conversion layer 104. When the photoelectric conversion film has no auxiliary layer, the photoelectric conversion layer 104 is the organic thin film described above. When the photoelectric conversion film has an auxiliary layer, at least one of the photoelectric conversion layer 104 and the auxiliary layer is the organic thin film described above. However, the auxiliary layer is preferably the organic thin film comprising the material for a photoelectric conversion element of the present embodiment from the viewpoint of more effectively and reliably exerting the working effects according to the present invention.

As one aspect of the present embodiment, a photoelectric conversion film 110 comprises a photoelectric conversion layer 104 and a second auxiliary layer 105 and a third auxiliary layer 107 positioned between the photoelectric conversion layer 104 and an upper electrode 106, wherein among the auxiliary layers 105 and 107, the third auxiliary layer 107, which is closer to the upper electrode 106, is adjacent to the upper electrode 106, and the second auxiliary layer 105 is positioned on the photoelectric conversion layer 104 side of the third auxiliary layer 107. The photoelectric conversion film 110 shown in Figure 2 comprises the first auxiliary layer 103, the second auxiliary layer 105, and the third auxiliary layer 107, while the photoelectric conversion film may comprise no auxiliary layer between a lower electrode 102 and the photoelectric conversion layer 104.

The photoelectric conversion layer 104 may be an organic semiconductor film generally used as a photoelectric conversion layer or may be the organic thin film described above. In the photoelectric conversion layer 110, the organic semiconductor film or the organic thin film may be one layer or a plurality of layers. In the case of one layer, p-type organic semiconductor film, a n-type organic semiconductor film, or a mixed film thereof (hereinafter, which may be "bulk-hetero structures") is used. On the other hand, in the case of a plurality of layers, the number of layers may be on the order of 2 to 10 layers. A structure is used where p-type organic semiconductor films, n-type organic semiconductor films, or mixed films thereof (hereinafter, which may be "bulk-hetero structures") are laminated. A buffer layer may be inserted between the layers.

The photoelectric conversion layer 104 according to the present embodiment may or may not comprise the material for a photoelectric conversion element of the present embodiment, and may comprise a material other than the material for a photoelectric conversion element of the present embodiment. Among others, the photoelectric conversion layer 104 preferably comprises at least one or more of organic p-type semiconductors, organic n-type semiconductors, and light-absorbing materials because incident light energy with a desired wavelength can be more efficiently converted to electric signals. Among others, an organic p-type semiconductor that easily donates electrons to the light-absorbing material and has a small ionization potential, or an organic n-type semiconductor that easily accepts electrons therefrom and has large electron affinity, is preferable because incident light energy can be still more efficiently converted to electric signals. In this context, the ionization potential (HOMO level) refers to a value measured by photoemission yield spectroscopy or photoelectron spectroscopy. The electron affinity (LUMO level) refers to a value determined by calculating an energy band gap value from the absorption end of the longest wavelength of near-infrared spectra, and subtracting the value from the HOMO level, or a value measured by inverse photoemission spectroscopy.

In the case of using an organic semiconductor film, the film may be one layer or may be two or more layers. The organic semiconductor film may be an organic p-type semiconductor film, may be an organic n-type semiconductor film, may be a light-absorbing material film, or may be a mixed film thereof (bulk-hetero structure). Particularly, the organic semiconductor film preferably has a layer having a bulk-hetero junction structure. In such a case, the photoelectric conversion film is allowed to have a bulk-hetero junction structure. This can compensate for the disadvantage, i.e., a short carrier diffusion length, of the photoelectric conversion film and improve photoelectric conversion efficiency.

The thickness of the photoelectric conversion layer 104 may be, for example, 0.5 nm or more and 5000 nm or less, may be 1 nm or more and 1000 nm or less, or may be 5 nm or more and 500 nm or less.

Hereinafter, the organic semiconductor will be described in detail.

The organic p-type semiconductor (compound) is a donor organic semiconductor (hereinafter, also referred to as a "donor organic compound") and refers to an organic compound that is typified mainly by a hole-transporting organic compound and has a property of easily donating electrons. Further specifically, this compound refers to an organic compound having a smaller ionization potential when two organic materials are used in contact. Thus, any organic compound may be used as the donor organic compound as long as the organic compound has electron-donating properties.

Examples of such a donor organic compound include triarylamine compounds, benzidine compounds, pyrazoline compounds, styrylamine compounds, hydrazone compounds, triphenylmethane compounds, carbazole compounds, polysilane compounds, thiophene compounds, phthalocyanine compounds, cyanine compounds, merocyanine compounds, oxonol compounds, polyamine compounds, indole compounds, pyrrole compounds, pyrazole compounds, polyarylene compounds, condensed aromatic carbocyclic compounds (e.g., naphthalene derivatives, anthracene derivatives, phenanthrene derivatives, tetracene derivatives, pyrene derivatives, perylene derivatives, and fluoranthene derivatives), and metal complexes having nitrogen-comprising heterocyclic compounds as ligands. The donor organic compound is not limited thereto, and as described above, an organic compound having a smaller ionization potential than that of an organic compound used as an acceptor organic compound may be used as the donor organic semiconductor.

The organic n-type semiconductor (compound) is an acceptor organic semiconductor (hereinafter, also referred to as an "acceptor organic compound") and refers to an organic compound that is typified mainly by an electron-transporting organic compound and has a property of easily accepting electrons. Further specifically, this compound refers to an organic compound having larger electron affinity when two organic compounds are used in contact. Thus, any organic compound may be used as the acceptor organic compound as long as the organic compound has electron-accepting properties.

Examples of such an acceptor organic compound include condensed aromatic carbocyclic compounds (e.g., naphthalene derivatives, anthracene derivatives, phenanthrene derivatives, tetracene derivatives, pyrene derivatives, perylene derivatives, fluoranthene derivatives, and fullerene derivatives), 5- to 7-membered heterocyclic compounds comprising nitrogen atom(s), oxygen atom(s), or sulfur atom(s) (e.g., pyridine, pyrazine, pyrimidine, pyridazine, triazine, quinoline, quinoxaline, quinazoline, phthalazine, cinnoline, isoquinoline, pteridine, acridine, phenazine, phenanthroline, tetrazole, pyrazole, imidazole, thiazole, oxazole, indazole, benzimidazole, benzotriazole, benzoxazole, benzothiazole, carbazole, purine, triazolopyridazine, triazolopyrimidine, tetrazaindene, oxadiazole, imidazopyridine, pyralidine, pyrrolopyridine, thiadiazolopyridine, dibenzazepine, and tribenzazepine), polyarylene compounds, fluorene compounds, cyclopentadiene compounds, silyl compounds, and metal complexes having nitrogen-comprising heterocyclic compounds as ligands. The acceptor organic compound is not limited thereto, and as described above, an organic compound having larger electron affinity than that of an organic compound used as a donor organic compound may be used as the acceptor organic semiconductor.

The light-absorbing material is a compound having a local maximum optical absorption wavelength in the visible light region, particularly, in the range of 450 nm or more and 650 nm or less. The absorption intensity in the local maximum optical absorption wavelength of the light-absorbing material is desirably larger than that in the local maximum optical absorption wavelength of a donor organic compound or an acceptor organic compound. The light-absorbing material having such absorption intensity can selectively absorb an incident light at the local maximum optical absorption wavelength. After an incident light is absorbed to the light-absorbing material so that photons become excitons, hole and electron carriers can be efficiently generated by causing exciton separation at the interface between a donor organic compound and an acceptor organic compound.

A compound generally called color can be used as such a light-absorbing material. Examples thereof include phthalocyanine derivatives, subphthalocyanine derivatives, quinacridone derivatives, porphyrin derivatives, naphthalene or perylene derivatives, phthaloperylene derivatives, styryl derivatives, cyanine derivatives, hemicyanine derivatives, merocyanine derivatives, rhodacyanine derivatives, oxonol derivatives, hemioxonol derivatives, croconium derivatives, squarylium derivatives, azamethine derivatives, arylidene derivatives, azo derivatives, azomethine derivatives, metallocene derivatives, fulgide derivatives, phenazine derivatives, phenothiazine derivatives, polyene derivatives, acridine derivatives, acridinone derivatives, diphenylamine derivatives, triarylamine derivatives such as triphenylamine, naphthylamine and styrylamine, quinophthalone derivatives, phenoxazine derivatives, chlorophyll derivatives, rhodamine derivatives, diphenylmethane or triphenylmethane derivatives, xanthene derivatives, acridine derivatives, phenoxazine derivatives, quinoline derivatives, oxazine derivatives, thiazine derivatives, quinone derivatives, benzoquinone derivatives, naphthoquinone derivatives, anthraquinone derivatives, indigo or thioindigo derivatives, pyrrole derivatives, pyridine derivatives, dipyrrin derivatives, indole derivatives, diketopyrrolopyrrole derivatives, coumarin derivatives, fluorene derivatives, fluorenone derivatives, fluoranthene derivatives, anthracene derivatives, pyrene derivatives, carbazole derivatives, phenylenediamine derivatives, benzidine derivatives, phenanthroline derivatives, imidazole derivatives, oxazoline derivatives, thiazoline derivatives, triazole derivatives, thiadiazole derivatives, oxazole derivatives, thiazole derivatives, oxadiazole derivatives, thiophene derivatives, selenophene derivatives, silole derivatives, germole derivatives, stilbene derivatives, phenylene vinylene derivatives, pentacene derivatives, rubrene derivatives, thienothiophene derivatives, benzodithiophene derivatives, xanthenoxanthene derivatives, and fullerene derivatives. The light-absorbing material is not limited thereto, and a compound having larger absorption intensity than that in the local maximum optical absorption wavelength of a donor organic compound or an acceptor organic compound can be used as the light-absorbing material, as described above. The light-absorbing material can also play a role as a donor organic compound or an acceptor organic compound.

As one aspect of the present embodiment, the first auxiliary layer 103 may be a single layer or may be two or more layers. The first auxiliary layer 103 has, for example, at least one of a hole blocking layer and an electron transport layer. When the first auxiliary layer 103 has two of these layers, the electron transport layer and the hole blocking layer are usually laminated in this order from the photoelectric conversion layer 104 side. The electron transport layer plays a role in transporting electrons generated in the photoelectric conversion layer 104 to the first electrode 102, and a role in blocking hole migration to the photoelectric conversion layer 104 from the first electrode 102 to which electrons are transported. The hole blocking layer plays a role in blocking hole migration from the first electrode 102 to the photoelectric conversion layer 104, preventing hole recombination in the photoelectric conversion layer 104, reducing dark current, reducing noise, and expanding a dynamic range. One layer may have the functions of both the hole blocking layer and the electron transport layer. The thickness of the first auxiliary layer 103 is preferably 10 nm or more and 300 nm or less, more preferably 30 nm or more and 250 nm or less, and further preferably 50 nm or more and 200 nm or less, from the viewpoint of suppressing dark current, and preventing reduction in photoelectric conversion efficiency.

As one aspect of the present embodiment, the second auxiliary layer 105 may be a single layer or may be two or more layers. The second auxiliary layer 105 has, for example, at least one of an electron blocking layer and a hole transport layer. When the second auxiliary layer 105 has two of these layers, the hole transport layer and the electron blocking layer are usually layered in this order from the photoelectric conversion layer 104 side. The hole transport layer plays a role in transporting generated holes from the photoelectric conversion layer 104 to the second electrode 106, and a role in blocking electron migration to the photoelectric conversion layer 104 from the second electrode 106 to which holes are transported. The electron blocking layer plays a role in blocking electron migration from the second electrode 106 to the photoelectric conversion layer 104, preventing electron recombination in the photoelectric conversion layer 104, reducing dark current, reducing noise, and expanding a dynamic range. One layer may have the functions of both the electron blocking layer and the hole transport layer. The thickness of the second auxiliary layer 105 is preferably 5 nm or more and 200 nm or less, more preferably 15 nm or more and 130 nm or less, and further preferably 25 nm or more and 100 nm or less, from the viewpoint of suppressing dark current, and preventing reduction in photoelectric conversion efficiency.

As one aspect of the present embodiment, the third auxiliary layer 107 is an auxiliary layer closer to the upper electrode 106 than the second auxiliary layer 105, and may be, for example, a hole blocking layer. The hole blocking layer plays a role in blocking hole migration from the second electrode 106 to the photoelectric conversion layer 104, preventing hole recombination in the photoelectric conversion layer 104, reducing dark current, reducing noise, and expanding a dynamic range. Note that at least one of the layers positioned between the photoelectric conversion layer 104 and the upper electrode 106 may have the functions of both the hole blocking layer and the electron transport layer. The thickness of the third auxiliary layer 107 is preferably 5 nm or more and 200 nm or less, more preferably 15 nm or more and 130 nm or less, and further preferably 25 nm or more and 100 nm or less, from the viewpoint of suppressing dark current, and preventing reduction in photoelectric conversion efficiency.

The material for a photoelectric conversion element of the present embodiment can be comprised in any of the first auxiliary layer 103, the second auxiliary layer 105, and the third auxiliary layer 107, and is preferably comprised in the first auxiliary layer 103 and/or the third auxiliary layer 107. In the photoelectric conversion element of the present embodiment, of the first auxiliary layer 103, the second auxiliary layer 105, and the third auxiliary layer 107, the first auxiliary layer 103 and the third auxiliary layer 107 preferably includes the organic thin film. The material for a photoelectric conversion element of the present embodiment is more preferably comprised in at least one of the hole blocking layer and the electron transport layer in the first auxiliary layer 103 and/or the third auxiliary layer 107. In the photoelectric conversion element of the present embodiment, at least one of the hole blocking layer and the electron transport layer is preferably the organic thin film. These can more effectively and reliably exert the working effects according to the present invention.

As one aspect of the present embodiment, the compounds (I) to (VI) of the present embodiment are comprised in, among these auxiliary layers, at least the third auxiliary layer 107. The third auxiliary layer 107 may comprise a material other than the compounds (I) to (VI). The content of the compounds (I) to (VI) in the third auxiliary layer 107 is not particularly limited as long as performance necessary for use as the auxiliary layer closer to the upper electrode 106 is exerted. The content thereof may be, for example, 50% by mass or more based on the total amount of the third auxiliary layer 107. However, from the viewpoint of more effectively and reliably exerting the working effects of suppressing leak current in the dark according to the present invention, the content thereof is preferably 80% by mass or more, more preferably 90% by mass or more, and further preferably 95% by mass or more. The upper limit of the content thereof may be 100% by mass.

Hereinafter, the material, other than the compounds (I) to (VI) and material for a photoelectric conversion element of the present embodiment, which may be comprised in each layer in the auxiliary layers, will be described.

The material for the hole transport layer is not particularly limited as long as it is known as a hole transport layer for photoelectric conversion elements such as solid image sensors. Examples thereof include polyaniline and doped materials thereof, and cyanogen compounds described in International Publication No. WO 2006/019270.

More specific examples of the material constituting the hole transport layer include selenium, iodides such as copper iodide (Cul), cobalt complexes such as layered cobalt oxide, CuSCN, molybdenum oxide (MoO₃, etc.), nickel oxide (NiO, etc.), 4CuBr·3S(C₄H₉) and organic hole transport materials. Among them, examples of the iodide include copper iodide (Cul). Examples of the layered cobalt oxide include AxCoO₂ (wherein A represents Li, Na, K, Ca, Sr or Ba, and 0 ≤ X ≤ 1). Examples of the organic hole transport material include polythiophene derivatives such as poly-3-hexylthiophene (P3HT) and poly(3,4-ethylenedioxythiophene) (PEDOT; e.g., trade name "BaytronP" manufactured by H.C. Starck-V Tech Ltd.), fluorene derivatives such as 2,2',7,7'-tetrakis-(N,N-di-p-methoxyphenylamine)-9,9'-spirobifluorene (spiro-MeO-TAD), carbazole derivatives such as polyvinylcarbazole, triphenylamine derivatives, diphenylamine derivatives, polysilane derivatives, and polyaniline derivatives. Further examples of the material for the hole transport layer include compound semiconductors having monovalent copper, such as CuInSe₂ and copper sulfide (CuS), gallium phosphide (GaP), nickel oxide (NiO), cobalt oxide (CoO), iron oxide (FeO), bismuth oxide (Bi₂O₃), molybdenum oxide (MoO₂), and chromium oxide (Cr₂O₃).

The hole transport layer having a higher LUMO level than that of the photoelectric conversion film is preferred because an electron blocking function having a rectifying effect of suppressing the migration of electrons generated in the photoelectric conversion film to the electrode side is imparted thereto. Such a hole transport layer is also called an electron blocking layer.

Examples of the low-molecular organic compound as the material constituting the electron blocking layer include aromatic diamine compounds such as N,N'-bis(3-methylphenyl)-(1,1'-biphenyl)-4,4'-diamine (TPD) and 4,4'-bis[N-(naphthyl)-N-phenyl-amino]biphenyl (α-NPD), oxazole, oxadiazole, triazole, imidazole, imidazolone, stilbene derivatives, pyrazoline derivatives, tetrahydroimidazole, polyarylalkane, butadiene, 4,4',4"-tris(N-(3-methylphenyl)N-phenylamino)triphenylamine (m-MTDATA), porphyrin compounds such as porphyrin, copper tetraphenylporphyrin, phthalocyanine, copper phthalocyanine and titanium phthalocyanine oxide, triazole derivatives, oxadiazole derivatives, imidazole derivatives, polyarylalkane derivatives, pyrazoline derivatives, pyrazolone derivatives, phenylenediamine derivatives, arylamine derivatives, amino-substituted chalcone derivatives, oxazole derivatives, styrylanthracene derivatives, fluorenone derivatives, hydrazone derivatives, and silazanes derivatives. Examples of the high-molecular organic compound include polymers such as phenylene vinylene, fluorene, carbazole, indole, pyrene, pyrrole, picoline, thiophene, acetylene and diacetylene, and derivatives thereof. A compound having sufficient hole-transporting properties, albeit being not an electron-donating compound, may be used as the material constituting the electron blocking layer. Examples of the inorganic compound as the material constituting the electron blocking layer include metal oxides such as calcium oxide, chromium oxide, copper chromium oxide, manganese oxide, cobalt oxide, nickel oxide, copper oxide, copper gallium oxide, copper strontium oxide, niobium oxide, molybdenum oxide, copper indium oxide, silver indium oxide and iridium oxide, selenium, tellurium and antimony sulfide. These materials may each be used singly or in combination of two or more thereof.

The thickness of the hole transport layer is preferably 10 nm or more and 300 nm or less, more preferably 30 nm or more and 250 nm or less, further preferably 50 nm or more and 200 nm or less, from the viewpoint of suppressing dark current, and preventing reduction in photoelectric conversion efficiency.

The method for forming the hole transport layer and the electron blocking layer may be a heretofore known method and may be any of dry film formation methods such as vacuum vapor deposition methods, and wet film formation methods such as solution coating methods. A wet film formation method is preferred from the viewpoint of being able to level a coated surface. Examples of the dry film formation method include vapor deposition methods such as vacuum vapor deposition methods, and sputter methods. The vapor deposition may be any of physical vapor deposition (PVD) and chemical vapor deposition (CVD) and is preferably physical vapor deposition such as vacuum vapor deposition. Examples of the wet film formation method include inkjet methods, spray methods, nozzle print methods, spin coating methods, dip coating methods, casting methods, die coating methods, roll coating methods, bar coating methods and gravure coating methods.

The material constituting the electron transport layer is not particularly limited as long as it is known as an electron transport layer for photoelectric conversion elements such as solid image sensors. Examples thereof include organic compounds such as perfluoro forms (e.g., perfluoropentacene, perfluorophthalocyanine, etc.) of octaazaporphyrin and p-type semiconductors, fullerene, fullerene derivatives (e.g., [6,6]-phenyl-C61-butyric acid methyl ester; PCBM, etc.), perylene, indenoindene and indenoindene derivatives, and inorganic oxides such as titanium oxide (TiO₂, etc.), nickel oxide (NiO), tin oxide (SnO₂), tungsten oxide (WO₂, WO₃, W₂O₃, etc.), zinc oxide (ZnO), niobium oxide (Nb₂O₅, etc.), tantalum oxide (Ta₂O₅, etc.), yttrium oxide (Y₂O₃, etc.), and strontium titanate (SrTiO₃, etc.). The electron transport layer may be a porous layer or may be a dense layer. In the case of laminating them, the porous electron transport layer and the dense electron transport layer are preferably laminated and disposed in this order from the photoelectric conversion film side.

The electron transport layer having a lower HOMO level than that of the photoelectric conversion film is preferred because a hole blocking function having a rectifying effect of suppressing the migration of holes generated in the photoelectric conversion film to the opposite electrode side is imparted thereto. Such an electron transport layer is also called a hole blocking layer.

Examples of the material constituting the hole blocking layer include oxadiazole derivatives such as 1,3-bis(4-tert-butylphenyl-1,3,4-oxadiazolyl)phenylene (OXD-7), anthraquinone dimethane derivatives, diphenylquinone derivatives, bathocuproine, bathophenanthroline, and their derivatives, triazine compounds, triazole compounds, tris(8-hydroxyquinolinato)aluminum complexes, bis(4-methyl-8-quinolinato)aluminum complexes, silole compounds, porphyrin compounds, styryl compounds such as DCM (4-dicyanomethylene-2-methyl-6-(4-(dimethylaminostyryl))-4H-pyran), n-type semiconductor materials such as naphthalenetetracarboxylic anhydride (NTCDA), naphthalenetetracarboxylic acid diimide, perylenetetracarboxylic anhydride (PTCDA), and perylenetetracarboxylic acid diimide, n-type inorganic oxides such as titanium oxide, zinc oxide and gallium oxide, and alkali metal fluorides such as lithium fluoride, sodium fluoride and cesium fluoride. Further, an organic semiconductor molecule doped with an alkali metal compound is also preferred because of having a function of improving electric junction to the opposite electrode. These materials may each be used singly or in combination of two or more thereof.

The thickness of the electron transport layer is preferably 10 nm or more and 300 nm or less, more preferably 30 nm or more and 250 nm or less, further preferably 50 nm or more and 200 nm or less, from the viewpoint of suppressing dark current, and preventing reduction in photoelectric conversion efficiency.

The method for forming the electron transport layer and the hole blocking layer may be a heretofore known method and may be any of dry film formation methods such as vacuum vapor deposition methods, and wet film formation methods such as solution coating methods. A wet film formation method is preferred from the viewpoint of being able to level a coated surface. Examples of the dry film formation method include vapor deposition methods such as vacuum vapor deposition methods, and sputter methods. The vapor deposition may be any of physical vapor deposition (PVD) and chemical vapor deposition (CVD) and is preferably physical vapor deposition such as vacuum vapor deposition. Examples of the wet film formation method include inkjet methods, spray methods, nozzle print methods, spin coating methods, dip coating methods, casting methods, die coating methods, roll coating methods, bar coating methods and gravure coating methods.

The photoelectric conversion element of the present embodiment may have single or two or more auxiliary layers other than the first auxiliary layer 103 between the first auxiliary layer 103 and the lower electrode 102. Examples of such an auxiliary layer include hole injection layers that improve hole injectability from the lower electrode 102 to the first auxiliary layer 103. Examples of the material constituting the hole injection layer include phthalocyanine derivatives, starburst amines such as m-MTDATA (4,4',4"-tris[phenyl(m-tolyl)amino]triphenylamine), and polymer materials including polythiophene and polyvinylcarbazole derivatives, such as PEDOT (poly(3,4-ethylenedioxythiophene)). The thickness of this auxiliary layer may be the same as that of the first auxiliary layer 103.

The photoelectric conversion element of the present embodiment may have single or two or more auxiliary layers other than the second auxiliary layer 105 between the second auxiliary layer 105 and the upper electrode 106. Examples of such an auxiliary layer include electron injection layers that improve electron injectability from the upper electrode 106 to the second auxiliary layer 105, and electron transport layers. Examples of the material constituting the electron injection layer include metals such as cesium, lithium and strontium, and lithium fluoride. The material constituting the electron transport layer may be the same as described above. The thickness of this auxiliary layer may be the same as that of the second auxiliary layer 105.

The photoelectric conversion element of the present embodiment may have single or two or more auxiliary layers other than the second auxiliary layer 105 and the third auxiliary layer 107 between the third auxiliary layer 107 and the upper electrode 106. Examples of such an auxiliary layer include electron injection layers that improve electron injectability from the upper electrode 106 to the third auxiliary layer 107, and electron transport layers. Examples of the material constituting the electron injection layer include metals such as cesium, lithium and strontium, and lithium fluoride. The material constituting the electron transport layer may be the same as described above. The thickness of this auxiliary layer may be the same as that of the second auxiliary layer 105.

The photoelectric conversion element of the present embodiment may have, in addition to each of the layers mentioned above, at least one of an interlayer contact improvement layer and a crystallization prevention layer positioned between the layers.

The interlayer contact improvement layer has a function of reducing damage on a lower film nearest to an upper electrode, for example, the photoelectric conversion film 110, at the time of film formation of the upper electrode 106. Particularly, high-energy particles present in an apparatus for use in the film formation of the upper electrode 106 to be formed, for example, sputter particles, secondary electrons, Ar particles, or oxygen anions in a sputtering method, may deteriorate the lower film nearest thereto through collision, resulting in performance deterioration such as increased leak current or reduced sensitivity. One of the methods for preventing this preferably involves establishing an interlayer contact improvement layer on an upper layer of the nearest lower film. An organic material such as copper phthalocyanine, NTCDA, PTCDA, [dipyrazino[2,3-F:2',3'-H]quinoxaline-2,3,6,7,10,11-hexacarbonitrile] (HATCN), an acetyl acetonate complex, or BCP, an organic metal compound, or an inorganic material such as MgAg or MgO is preferably used as the material for the interlayer contact improvement layer. The thickness of the interlayer contact improvement layer differs in proper range depending on the configuration of a photoelectric conversion film, the film thickness of an electrode, etc. It is particularly preferred to select a material having no absorption in the visible region, or the thickness is preferably 2 nm or more and 500 nm from the viewpoint of using a small thickness.

As mentioned above, the photoelectric conversion element of the present embodiment is connected with an accumulation unit serving as a condenser for accumulation of the generated charge and a readout unit serving as a transistor circuit for readout via a connection unit made of a conductive material. Also, the photoelectric conversion element optionally comprises a protective structure against outside air, such as a protective film, a substrate for strength retention, a microlens for light collection, or the like.

The readout unit is disposed in order to read out signals depending on a charge generated in the photoelectric conversion film. The readout unit is constituted by, for example, CCD, a CMOS circuit, or a TFT circuit, and preferably shielded from lights by a light shielding layer disposed in an insulating layer. The readout circuit is electrically connected with its corresponding electrode via a connection unit. In order to secure a charge in a quantity necessary for readout, an accumulation unit constituted by a condenser or the like may intervene between the electrode and the connection unit. The connection unit is embedded in the insulating layer and is a plug or the like for electrically connecting the electrode (e.g., a transparent electrode or an opposite electrode) to the readout unit. When the member thus configured is a solid image sensor, upon light incidence, the light is incident on the photoelectric conversion film where a charge is then generated. Electrons in the generated charge are collected (and accumulated) in one electrode, and holes are collected in the other electrode. Voltage signals depending on the quantity thereof is output to the outside of the solid image sensor by the readout unit.

### (Image sensor)

As one aspect of the present embodiment, the image sensor of the present embodiment may have the same configuration as that of a conventional image sensor except that the image sensor has the photoelectric conversion element of the present embodiment. The image sensor of the present embodiment has, for example, a large number of photoelectric conversion elements of the present embodiment disposed in an array pattern. Specifically, a large number of photoelectric conversion elements disposed in an array pattern constitute a solid image sensor that exhibits the quantity of an incident light as well as positional information on incidence.

The image sensor of the present embodiment may have one photoelectric conversion element of the present embodiment or may be prepared by laminating two or more photoelectric conversion elements of the present embodiment. In the case of laminating two or more photoelectric conversion elements of the present embodiment, the respective photoelectric conversion elements may perform photoelectric conversion by selectively detecting lights at wavelength bands different from each other. In the case of laminating three or more photoelectric conversion elements of the present embodiment, at least one thereof obtains green color signals, at least one of the remaining photoelectric conversion elements obtains blue color signals, at least one of the remaining photoelectric conversion elements obtains red color signals, and at least one of the remaining photoelectric conversion elements obtains color signals of infrared light. The image sensor can thereby obtain plural types of color signals by one pixel without the use of a color filter. Color signals other than those to be detected by the photoelectric conversion elements of the present embodiment may be sensed by a device having a heretofore known silicon photodiode.

In the image sensor, a plurality of photoelectric conversion elements and a device having a silicon photodiode may be laminated as long as a photoelectric conversion element disposed nearer a light source does not block (i.e., transmissive to) the absorption wavelength of another photoelectric conversion element disposed at the back thereof viewed from the light source side.

In the image sensor, the photoelectric conversion elements may be partially constituted as a thin film in the same plane having no structural separation between the adjacent photoelectric conversion elements, from the viewpoint of easy molding.

The image sensor of the present embodiment may further include a substrate. The substrate is used for producing the image sensor by laminating each layer thereon, or used for enhancing the mechanical strength of the image sensor. Examples of the type of the substrate include, but are not particularly limited to, semiconductor substrates, glass substrates and plastic substrates.

In the present embodiment, the image sensor comprises the compound (VI), compound (II), compound (III), or compound (IV), and therefore, there can be provided an image sensor that can suppress leak current in the dark and has excellent wavelength selectivity. Accordingly, the compound (VI), compound (II), compound (III), or compound (IV) can suppress charge separation due to noise or light of other wavelengths, and is suitable for an image sensor requiring high resolution. Moreover, although not particularly limited, the compound (VI), compound (II), compound (III), or compound (IV) is preferably comprised in the photoelectric conversion film of the image sensor, and more preferably comprised in the auxiliary layer closer to the second electrode film, from the viewpoint of more effectively and reliably exerting the working effects according to the present invention. Here, the photoelectric conversion film and the auxiliary layer closer to the second electrode film are as described above.

### (Photosensor)

The photosensor of the present embodiment may have the same configuration as that of a conventional photosensor except that the photosensor has the image sensor of the present embodiment. This photosensor receives a light by the image sensor of the present embodiment and can output electric signals in response to the amount of the light received.

### (Solid-state imaging apparatus)

The solid-state imaging apparatus of the present embodiment may have the same configuration as that of a conventional solid-state imaging apparatus except that the solid-state imaging apparatus has the image sensor of the present embodiment. The solid-state imaging apparatus of the present embodiment may be, for example, a CMOS image sensor, and may have a pixel unit as an imaging area on a semiconductor substrate and further have a peripheral circuit unit having a row scanning unit, a horizontal selection unit, a column scanning unit, and a system control unit, in a peripheral region of or beneath in a vertical direction this pixel unit. The pixel unit has the image sensor of the present embodiment.

The photoelectric conversion element of the present embodiment employs the material for a photoelectric conversion element of the present embodiment and thereby has the following advantages: the photoelectric conversion element of the present embodiment has a low dark current value because a short circuit or a pinhole is unlikely to occur. As a result, the photoelectric conversion element of the present embodiment has excellent leak-preventing properties (particularly, in the dark). Moreover, the photoelectric conversion element of the present embodiment tends to easily exhibit a high light/dark ratio and in this case, has better leak-preventing properties. Also, the photoelectric conversion element of the present embodiment is excellent in hole- and electron-transporting properties, despite the material for a photoelectric conversion element that is unlikely to aggregate, and therefore has high photoelectric conversion efficiency. Besides, the photoelectric conversion element of the present embodiment employs the material for a photoelectric conversion element of the present embodiment and thereby has favorable heat resistance, and its durability in a production process and an environment of actual use is improved.

### Examples

Hereinafter, the present invention will be described in more detail with reference to Examples. However, the present invention is not limited by these Examples. Synthesized compounds were further purified by sublimation, if necessary.

### <Synthesis Example 1>

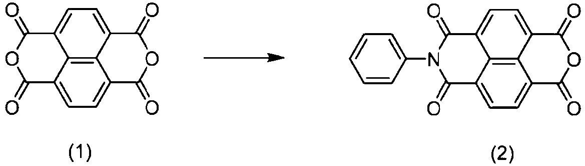

A mixture obtained by adding 6.0 g of 1,4,5,8-naphthalenetetracarboxylic dianhydride (hereinafter, also referred to as a "compound (1)") (manufactured by Tokyo Chemical Industry Co., Ltd.) and 2.1 g (1.0 molar equivalent based on the compound (1)) of aniline (manufactured by Tokyo Chemical Industry Co., Ltd.) to 90 mL of N,N-dimethylformamide (manufactured by Tokyo Chemical Industry Co., Ltd.) was stirred at 150°C for 8 hours. Then, the mixture was cooled to room temperature, and the solvent was distilled off under reduced pressure. Acetone was added to the solid obtained by distilling off the solvent, and water was gradually added thereto with stirring. A deposited mixture was filtered. After dissolution of the deposited mixture in chloroform, sodium sulfate was added thereto, and the mixture was left standing for 30 minutes. After subsequent filtration of sodium sulfate, the solvent was distilled off under reduced pressure. A pale yellow solid compound (2) was obtained by size exclusion chromatography using chloroform as an eluent.

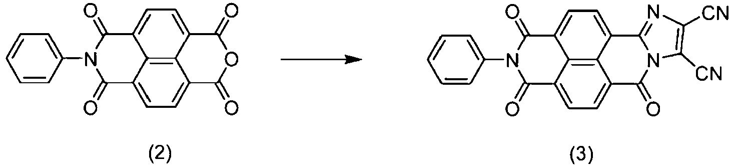

A mixture obtained by adding 3.0 g of the compound (2), 1.4 g (1.5 molar equivalent based on the compound (2)) of diaminomaleonitrile (manufactured by Tokyo Chemical Industry Co., Ltd.), and 1.6 g (1.5 molar equivalent based on the compound (2)) of benzoic acid (manufactured by Tokyo Chemical Industry Co., Ltd.) to 50 mL of N-methylpyrrolidone (manufactured by Tokyo Chemical Industry Co., Ltd.) was stirred at 200°C for 8 hours. Then, the mixture was cooled to room temperature, and methanol was added thereto. A deposited mixture was filtered. An orange solid compound (3) was obtained by size exclusion chromatography using chloroform as an eluent. Results of NMR measurement thereof are shown below. ¹HNMR (500 MHz, DMSO-d6): 8.85 (dd, 2H), 8.76 (dd, 2H), 7.56 (dm, 2H), 7.51 (dm, 1H), 7.45 (d, 2H)

### <Synthesis Example 2>

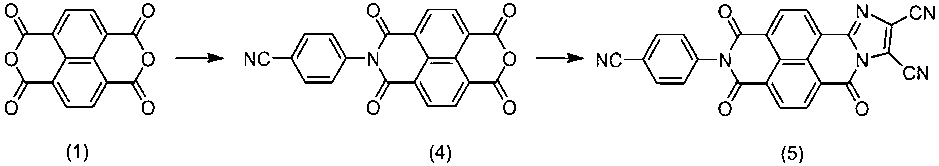

A compound (5) was obtained in the same manner as in Synthesis Example 1 except that 4-aminobenzonitrile (manufactured by Tokyo Chemical Industry Co., Ltd.) was used instead of aniline. Results of NMR measurement thereof are shown below.
¹HNMR (500 MHz, DMSO-d6): 8.96 (dd, 2H), 8.77 (dd, 2H), 8.04 (d, 2H), 7.63 (d, 2H)

### <Synthesis Example 3>

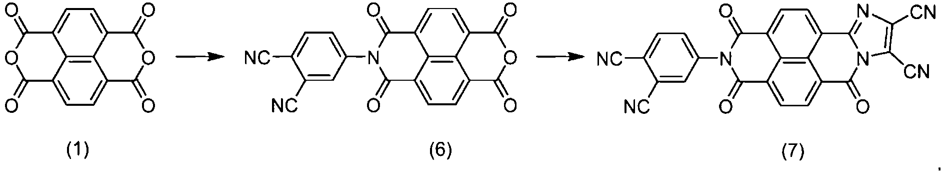

A compound (7) was obtained in the same manner as in Synthesis Example 1 except that 4-aminophthalonitrile (manufactured by Tokyo Chemical Industry Co., Ltd.) was used instead of aniline. Results of NMR measurement thereof are shown below.
¹HNMR (500 MHz, DMSO-d6): 9.01 (d, 1H), 8.94 (d, 1H), 8.82 (d, 1H), 8.78 (d, 1H), 8.37 (d, 1H), 8.30 (d, 1H), 8.09 (dd, 1H)

### <Synthesis Example 4>

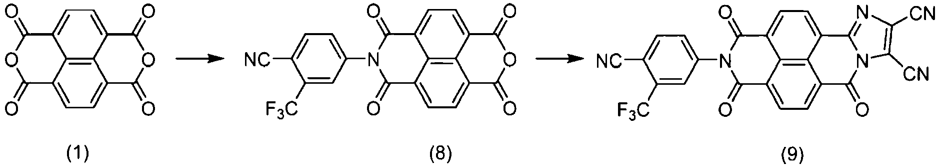

A compound (9) was obtained in the same manner as in Synthesis Example 1 except that 4-cyano-3-trifluoromethylaniline (manufactured by Tokyo Chemical Industry Co., Ltd.) was used instead of aniline. Results of NMR measurement thereof are shown below.
¹HNMR (500 MHz, DMSO-d6): 9.01 (d, 1H), 8.93 (d, 1H), 8.79 (dd, 2H), 8.44 (d, 1H), 8.26 (d, 1H), 8.07 (dd, 1H)

### <Synthesis Example 5>

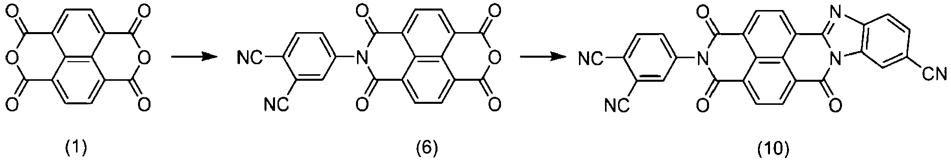

A compound (10) was obtained in the same manner as in Synthesis Example 3 except that 3,4-diaminobenzonitrile (manufactured by Tokyo Chemical Industry Co., Ltd.) was used instead of diaminomaleonitrile. Results of NMR measurement thereof are shown below.
¹HNMR (500 MHz, DMSO-d6): 9.01 (d, 1H), 8.95 (d, 1H), 8.80 (s, 3H), 8.40 (d, 1H), 8.34 (s, 1H), 8.13 (m, 2H), 7.98 (d, 1H)

### <Synthesis Example 6>

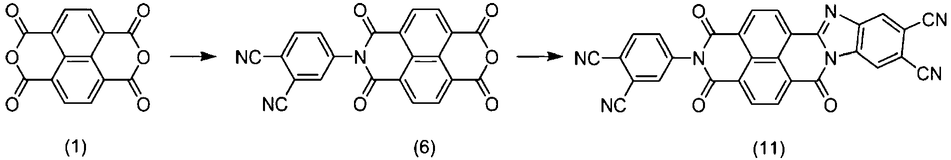

A compound (11) was obtained in the same manner as in Synthesis Example 3 except that 4,5-diaminophthalonitrile (manufactured by Tokyo Chemical Industry Co., Ltd.) was used instead of diaminomaleonitrile. Results of NMR measurement thereof are shown below.
¹HNMR (500 MHz, DMSO-d6): 9.06 (s, 2H), 8.98 (s, 1H), 8.87 (s, 1H), 8.80 (s, 2H), 8.40 (d, 1H), 8.33 (2, 1H), 8.11 (dd, 1H)

### <Synthesis Example 7>

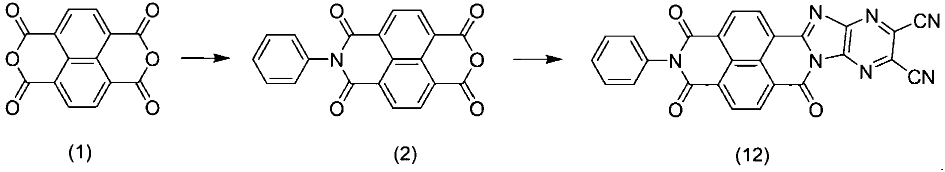

A compound (12) was obtained in the same manner as in Synthesis Example 1 except that 5,6-diamino-2,3-dicyanopyrazine (manufactured by Tokyo Chemical Industry Co., Ltd.) was used instead of diaminomaleonitrile. Results of NMR measurement thereof are shown below.
¹HNMR (500 MHz, DMSO-d6): 9.22 (d, 1H), 9.02 (d, 1H), 8.82 (d, 2H), 7.58-7.47 (m, 5H)

### <Synthesis Example 8>

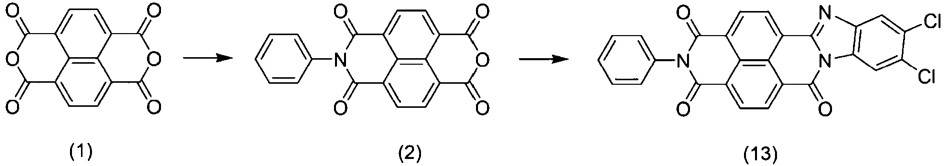

A compound (13) was obtained in the same manner as in Synthesis Example 1 except that 4,5-dichloro-1,2-phenylenediamine (manufactured by Tokyo Chemical Industry Co., Ltd.) was used instead of diaminomaleonitrile. Results of NMR measurement thereof are shown below.
¹HNMR (500 MHz, TFA-d): 9.23 (dd, 2H), 9.07 (dd, 2H), 8.95 (s, 1H), 8.12 (s, 1H), 7.57 (dd, 3H), 7.32 (m, 1H)

### <Synthesis Example 9>

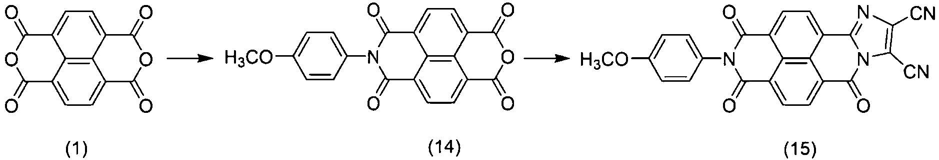

A compound (15) was obtained in the same manner as in Synthesis Example 1 except that p-anisidine (manufactured by Tokyo Chemical Industry Co., Ltd.) was used instead of aniline. Results of NMR measurement thereof are shown below.
¹HNMR (500 MHz, DMSO-d6): 8.99 (d, 1H), 8.91 (d, 1H), 8.75 (dd, 2H), 7.34 (d, 2H), 7.09 (d, 2H), 3.84 (s, 3H)

### <Synthesis Example 10>

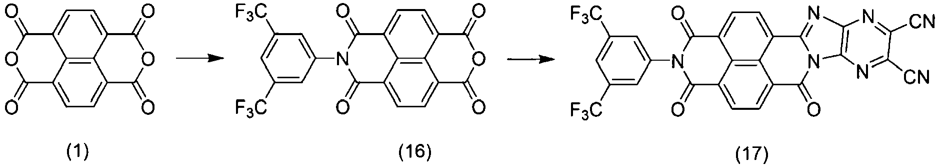

A compound (17) was obtained in the same manner as in Synthesis Example 7 except that 3,5-bis(trifluoromethyl)aniline (manufactured by Tokyo Chemical Industry Co., Ltd.) was used instead of aniline. Results of NMR measurement thereof are shown below.
¹HNMR (500 MHz, DMSO-d6): 9.26 (d, 1H), 9.05 (d, 1H), 8.86 (d, 2H), 8.37 (s, 1H), 8.36 (s, 2H)

### <Synthesis Example 11>

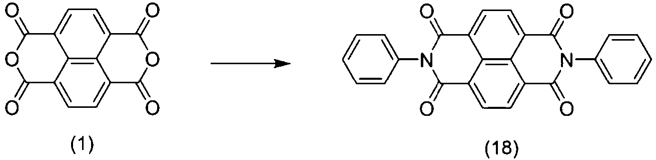

A mixture obtained by adding 2.0 g of the compound (1) (manufactured by Tokyo Chemical Industry Co., Ltd.) and 1.5 g (2.2 molar equivalent based on the compound (1)) of aniline (manufactured by Tokyo Chemical Industry Co., Ltd.) to 15 mL of acetic acid (manufactured by Tokyo Chemical Industry Co., Ltd.) was stirred under reflux at 125°C for 8 hours. Then, the mixture was cooled to room temperature, and methanol was added thereto. A deposited mixture was filtered. The mixture was further washed with methanol. Then, pyridine was added thereto, and the mixture was stirred for 5 minutes. Subsequently, the mixture was filtered, washed with methanol, and subjected to sublimation and purification to obtain a white solid compound (18). Results of NMR measurement thereof are shown below.
¹HNMR (500 MHz, DMSO-d6): 8.73 (s, 4H), 7.58-7.45 (m, 10H)

### <Synthesis Example 12>

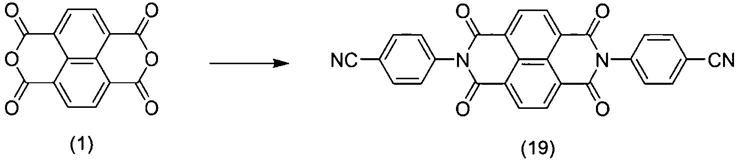

A mixture obtained by adding 6.0 g of the compound (1) (manufactured by Tokyo Chemical Industry Co., Ltd.), 6.1 g (2.1 molar equivalent based on the compound (1)) of isoquinoline (manufactured by Tokyo Chemical Industry Co., Ltd.), and 6.6 g (2.5 molar equivalent based on the compound (1)) of 4-aminobenzonitrile (manufactured by Tokyo Chemical Industry Co., Ltd.) to 90 mL of m-cresol (manufactured by Tokyo Chemical Industry Co., Ltd.) was stirred at 180°C for 8 hours. Then, the mixture was cooled to room temperature, and methanol was added thereto. A deposited mixture was filtered. After further washing with methanol, an aqueous potassium carbonate solution was added thereto, and the mixture was stirred for 5 minutes. Subsequently, the mixture was filtered, then washed with water, methanol, and purified by sublimation to obtain a yellow solid compound (19). Results of NMR measurement thereof are shown below. ¹HNMR (500 MHz, HFIP-d2): 8.93 (s, 4H), 8.77 (dm, 4H), 7.55 (dm, 4H)

### <Synthesis Example 13>

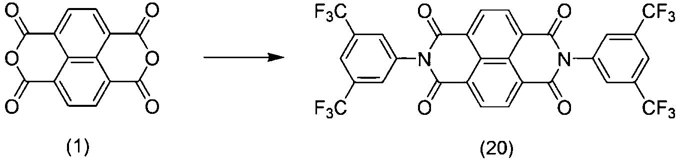

A compound (20) was obtained in the same manner as in Synthesis Example 11 except that 3,5-bis(trifluoromethyl)aniline (manufactured by Tokyo Chemical Industry Co., Ltd.) was used instead of aniline. Results of NMR measurement thereof are shown below.
¹HNMR (500 MHz, HFIP-d2): 8.54 (s, 4H), 7.81 (s, 2H), 7.52 (s, 4H)

### <Synthesis Example 14>

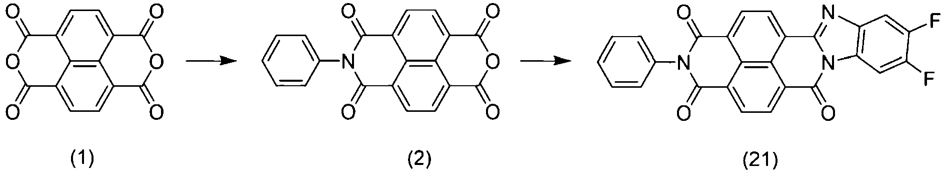

A compound (21) was obtained in the same manner as in Synthesis Example 1 except that 4,5-difluoro-1,2-phenylenediamine (manufactured by Tokyo Chemical Industry Co., Ltd.) was used instead of diaminomaleonitrile. Results of NMR measurement thereof are shown below.
¹HNMR (500 MHz, DMSO-d6): 8.93 (dd, 2H), 8.76 (dd, 2H), 8.41 (dd, 1H), 8.12 (dd, 1H), 7.58-7.46 (m, 5H)

### <Synthesis Example 15>

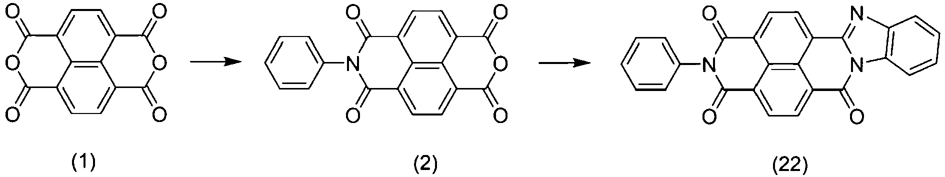

A compound (22) was obtained in the same manner as in Synthesis Example 1 except that 1,2-phenylenediamine (manufactured by Tokyo Chemical Industry Co., Ltd.) was used instead of diaminomaleonitrile. Results of NMR measurement thereof are shown below.
¹HNMR (500 MHz, DMSO-d6): 9.22 (t, 2H), 9.07 (dd, 2H), 8.83 (d, 1H), 7.98 (d, 1H), 7.90-7.83 (m, 2H), 7.57 (dd, 3H), 7.34 (m, 2H)

### <Synthesis Example 16>

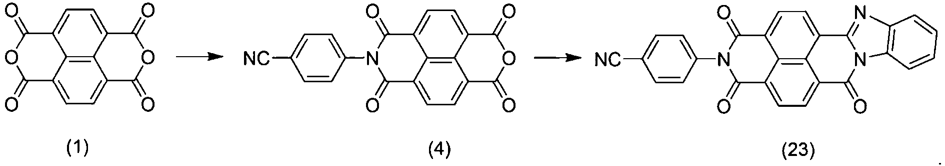

A compound (23) was obtained in the same manner as in Synthesis Example 2 except that 1,2-phenylenediamine (manufactured by Tokyo Chemical Industry Co., Ltd.) was used instead of diaminomaleonitrile. Results of NMR measurement thereof are shown below.
¹HNMR (500 MHz, DMSO-d6): 9.23 (dd, 2H), 9.07 (dd, 2H), 8.82 (d, 1H), 7.99-7.95 (m, 3H), 7.91-7.83 (m, 2H), 7.58 (d, 2H)

### [Method for Measuring Mass Reduction Temperature of Compound]

The mass reduction temperature (°C) when heating the compound from 20°C to 550°C under a pressure of 101325 Pa, at nitrogen of 200 mL/min, and at a temperature increase rate of 10°C/min was measured by the following method. Each weighed compound was placed in an aluminum pan, the pan was set at a predetermined position of a simultaneous thermogravimetric and differential thermal analyzer (STA 7200, Hitachi High-Technologies Corporation), and the 5% mass reduction temperature when heated from 20°C to 550°C under a pressure of 101325 Pa, at nitrogen of 200 mL/min, and at a temperature increase rate of 10°C/min was measured. The results are shown in Table 2.

The mass reduction temperature (^{O}C) when heating the compound from 20°C to 450°C under a pressure of 1 Pa and at a temperature increase rate of 10°C/min was measured by the following method. Each weighed compound was placed in an aluminum pan, the pan was set at a predetermined position of a vacuum thermogravimetric and differential thermal analyzer (STA 2500 Regulus (trade name), NETZSCH Japan K.K.), and the 10% mass reduction temperature when heated from 20°C to 450°C under a pressure of 1 Pa and at a temperature increase rate of 10°C/min was measured. The results are shown in Table 2.

### [Preparation and evaluation of organic thin film and photoelectric conversion element]

In Examples and Comparative Examples given below, an organic thin film and a photoelectric conversion element were prepared using a vapor deposition machine, and the application and measurement of current and voltage were performed in air. The prepared photoelectric conversion element was placed in a measurement chamber, and the application and measurement of current and voltage were performed. The application and measurement of current and voltage employed an automatic IV measuring device (manufactured by System Engineers Co., Ltd.). Irradiation with incident light was performed under conditions involving an irradiation light wavelength of 550 nm and an irradiation light half-value width of 20 nm using a light source apparatus (manufactured by Asahi Spectra Co., Ltd., product name (PVL-3300)). A light-dark ratio is a value determined by dividing a current value in the case of light irradiation by a current value in the dark.

### (Example 1)

On ITO transparent conductive glass (ITO manufactured by GEOMATEC Co., Ltd., thickness: 100 nm), a film of boron subphthalocyanine chloride (purified product manufactured by Sigma-Aldrich Co., LLC., purity > 99%) was formed in vacuum as a photoelectric conversion layer having a thickness of 100 nm. A film of a sublimation-purified product of tris(8-quinolinolato)aluminum (Alq3) (manufactured by Tokyo Chemical Industry Co., Ltd.) was formed thereon as an auxiliary layer 1 having a thickness of 25 nm by resistance heating vacuum vapor deposition, and a film of the compound (3) was then formed thereon as an auxiliary layer 2 having a thickness of 25 nm by resistance heating vacuum vapor deposition. Subsequently, on the auxiliary layer 2, a film of aluminum was formed in vacuum as an electrode having a thickness of 100 nm to prepare a photoelectric conversion element. A voltage of 4 V was applied to the obtained photoelectric conversion element using the ITO and aluminum as electrodes, and a current value in the dark and a current value at the time of light irradiation were measured. A light/dark ratio was calculated from the measurement results. The results are shown in Table 2. Note that the dark current value was expressed as a relative value with the value in Comparative Example 1 described later taken as 1, and evaluated as A when less than 1, as B when 1 or more and less than 10, and as C when 10 or more. The light/dark ratio was evaluated as A when 10 or more, as B when 1 or more and less than 10, and as C when less than 1. Moreover, the spectral sensitivity at wavelengths of 560 nm and 650 nm was measured for the obtained photoelectric conversion element. Specifically, under a condition in which a voltage of 4 V was applied to the photoelectric conversion element using ITO and aluminum as electrodes, the current value was measured when light having a wavelength of 560 nm or 650 nm, an irradiation light half-value width of 20 nm, and a constant light output intensity was irradiated. By using the obtained current value, the external quantum efficiency was calculated as the ratio of the number of electrons flowing through the element per unit time to the number of photons of the irradiated light per unit time, and this was taken as the spectral sensitivity. From the measured spectral sensitivity, the ratio of the spectral sensitivity at a wavelength of 560 nm to the spectral sensitivity at a wavelength of 650 nm was calculated, and evaluated as A when 10 or more, as B when 5 or more and less than 10, and as C when less than 5. The results are shown in Table 2.

### (Example 2)

A single-layer organic thin film and a photoelectric conversion element were prepared in the same manner as in Example 1 except that the compound (5) was used instead of the compound (3). The obtained photoelectric conversion element was evaluated in the same manner as in Example 1. The results are shown in Table 2.

### (Example 3)

A single-layer organic thin film and a photoelectric conversion element were prepared in the same manner as in Example 1 except that the compound (7) was used instead of the compound (3). The obtained photoelectric conversion element was evaluated in the same manner as in Example 1. The results are shown in Table 2.

### (Example 4)

A single-layer organic thin film and a photoelectric conversion element were prepared in the same manner as in Example 1 except that the compound (9) was used instead of the compound (3). The obtained photoelectric conversion element was evaluated in the same manner as in Example 1. The results are shown in Table 2.

### (Example 5)

A single-layer organic thin film and a photoelectric conversion element were prepared in the same manner as in Example 1 except that the compound (10) was used instead of the compound (3). The obtained photoelectric conversion element was evaluated in the same manner as in Example 1. The results are shown in Table 1.

### (Example 6)

A single-layer organic thin film and a photoelectric conversion element were prepared in the same manner as in Example 1 except that the compound (11) was used instead of the compound (3). The obtained photoelectric conversion element was evaluated in the same manner as in Example 1. The results are shown in Table 2.

### (Example 7)

A single-layer organic thin film and a photoelectric conversion element were prepared in the same manner as in Example 1 except that the compound (12) was used instead of the compound (3). The obtained photoelectric conversion element was evaluated in the same manner as in Example 1. The results are shown in Table 2.

### (Example 8)

A single-layer organic thin film and a photoelectric conversion element were prepared in the same manner as in Example 1 except that the compound (13) was used instead of the compound (3). The obtained photoelectric conversion element was evaluated in the same manner as in Example 1. The results are shown in Table 2.

### (Example 9)

A single-layer organic thin film and a photoelectric conversion element were prepared in the same manner as in Example 1 except that the compound (15) was used instead of the compound (3). The obtained photoelectric conversion element was evaluated in the same manner as in Example 1. The results are shown in Table 2.

### (Example 10)

A single-layer organic thin film and a photoelectric conversion element were prepared in the same manner as in Example 1 except that the compound (17) was used instead of the compound (3). The obtained photoelectric conversion element was evaluated in the same manner as in Example 1. The results are shown in Table 2.

### (Comparative Example 1)

A single-layer organic thin film and a photoelectric conversion element were prepared in the same manner as in Example 1 except that the compound (1) was used instead of the compound (3). The obtained photoelectric conversion element was evaluated in the same manner as in Example 1. The results are shown in Table 2.

### (Comparative Example 2)

A single-layer organic thin film and a photoelectric conversion element were prepared in the same manner as in Example 1 except that the compound (18) was used instead of the compound (3). The obtained photoelectric conversion element was evaluated in the same manner as in Example 1. The results are shown in Table 2.

### (Comparative Example 3)

A single-layer organic thin film and a photoelectric conversion element were prepared in the same manner as in Example 1 except that the compound (19) was used instead of the compound (3). The obtained photoelectric conversion element was evaluated in the same manner as in Example 1. The results are shown in Table 2.

### (Comparative Example 4)

A single-layer organic thin film and a photoelectric conversion element were prepared in the same manner as in Example 1 except that the compound (20) was used instead of the compound (3). The obtained photoelectric conversion element was evaluated in the same manner as in Example 1. The results are shown in Table 2.

### (Comparative Example 5)

A single-layer organic thin film and a photoelectric conversion element were prepared in the same manner as in Example 1 except that the compound (21) was used instead of the compound (3). The obtained photoelectric conversion element was evaluated in the same manner as in Example 1. The results are shown in Table 2.

### (Comparative Example 6)

A single-layer organic thin film and a photoelectric conversion element were prepared in the same manner as in Example 1 except that the compound (22) was used instead of the compound (3). The obtained photoelectric conversion element was evaluated in the same manner as in Example 1. The results are shown in Table 2.

### (Comparative Example 7)

A single-layer organic thin film and a photoelectric conversion element were prepared in the same manner as in Example 1 except that the compound (23) was used instead of the compound (3). The obtained photoelectric conversion element was evaluated in the same manner as in Example 1. The results are shown in Table 2.

**[Table 2]**

| | Compound | LUMO (eV) | Difference in energy level (eV) | 5 wt% reduction temperature (°C) | 10 wt% reduction temperature (°C) | Dark current value (relative value) | Light/dark ratio (relative value) | Spectral sensitivity ratio |
|---|---|---|---|---|---|---|---|---|
| | | DFT calculated value | DFT calculated value | TG-DTA measured value | Vacuum TG-DTA measured value | At time of application of 4 V | At time of application of 4 V | 560 nm/ 650 nm |
| Example 1 | (3) | -4.07 | 3.05 | 430 | 224 | A | A | A |
| Example 2 | (5) | -4.30 | 3.00 | 480 | 272 | A | A | A |
| Example 3 | (7) | -4.44 | 3.00 | 510 | 272 | A | A | A |
| Example 4 | (9) | -4.38 | 3.00 | 480 | 218 | A | A | A |
| Example 5 | (10) | -4.06 | 2.87 | 431 | 299 | A | A | A |
| Example 5 | (11) | -4.30 | 2.99 | 468 | 320 | A | A | A |
| Example 6 | (12) | -4.11 | 3.16 | 450 | 290 | A | A | A |
| Example 7 | (13) | -3.60 | 2.87 | 413 | 227 | A | A | A |
| Example 8 | (15) | -4.02 | 2.35 | 442 | 247 | A | A | A |
| Example 9 | (17) | -4.33 | 3.17 | 420 | 252 | A | A | A |
| Comparative Example 1 | (1) | -4.00 | 3.70 | 344 | 156 | B | B | C |
| Comparative Example 2 | (18) | -3.35 | 3.60 | 440 | 206 | C | C | C |
| Comparative Example 3 | (19) | -3.82 | 3.54 | 430 | 237 | C | C | C |
| Comparative Example 4 | (20) | -3.84 | 3.62 | 351 | 186 | C | C | C |
| Comparative Example 5 | (21) | -3.52 | 2.84 | 385 | 201 | A | A | B |
| Comparative Example 6 | (22) | -3.37 | 2.90 | 393 | 216 | C | C | C |
| Comparative Example 7 | (23) | -3.60 | 2.85 | 419 | 245 | A | B | C |

The results shown in Table 2 revealed that the photoelectric conversion element of the present invention exhibits a low dark current value and therefore has excellent leak-preventing properties, particularly in the dark. Particularly, in some Examples, the photoelectric conversion element of the present invention exhibited a high light-dark ratio and was therefore found to have much better leak-preventing properties. Furthermore, in Examples, the photoelectric conversion element of the present invention exhibited an excellent spectral sensitivity ratio and was therefore found to have high wavelength selectivity. From the above, it was found that the compounds (I) and (V) are suitable as materials for a photoelectric conversion element, particularly as materials comprised in an electron transport layer and a hole blocking layer of a photoelectric conversion element. Moreover, it was found that the compound (VI), compound (II), compound (III), and compound (IV) are suitable as materials comprised in an image sensor.

### Industrial Applicability

A photoelectric conversion element, image sensor, and the like comprising the compounds (I) to (VI) described above exhibit excellent leak-preventing properties and wavelength selectivity. Thus, the compound, material for a photoelectric conversion element, organic thin film, photoelectric conversion element, and image sensor of the present invention have industrial applicability in fields that require these characteristics. Specifically, the compound, material for a photoelectric conversion element, organic thin film, photoelectric conversion element, and image sensor of the present invention have industrial applicability as a solid image sensor in, for example, image sensors for security cameras, in-car cameras, cameras for uninhabited air vehicles, agricultural cameras, industrial cameras, medical cameras such as endoscopic cameras, cameras for game machines, digital still cameras, digital video cameras, cameras for mobile phones, and cameras for the other mobile instruments; image scanning elements for facsimiles, scanners and copying machines; and photosensors for biosensors and chemical sensors. Also, the compound, material for a photoelectric conversion element, organic thin film, photoelectric conversion element, and image sensor of the present invention have industrial applicability as a display using electroluminescence in, for example, television monitors, touch monitors, digital signages, wearable displays, electronic papers, and head-up displays for mobility application.

The present application is based on Japanese Patent Application No. 2023-088105 filed on May 29, 2023, the contents of which are incorporated herein by reference.

### Reference Signs List

100, 200 ... photoelectric conversion element, 101 ... base material, 102 ... lower electrode, 103 ... first auxiliary layer, 104 ... photoelectric conversion layer, 105 ... second auxiliary layer, 106 ... upper electrode, 107 ... third auxiliary layer, 110 ... photoelectric conversion film.

## Claims

1. A compound represented by the following formula (I): 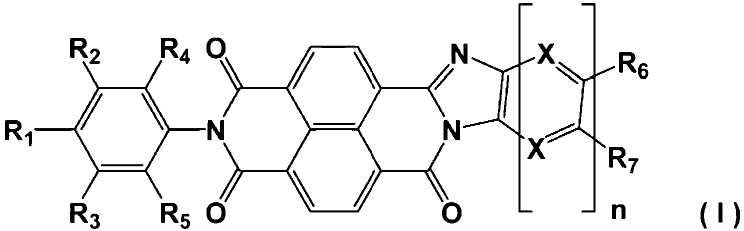 wherein
X is each independently selected from the group consisting of a methine group and a nitrogen atom;
n is an integer of 0 or more and 3 or less;
R₁, R₂, R₃, R₄, and R₅ are each independently a hydrogen atom, a halogen atom, or a monovalent organic group, and one or more of R₁, R₂, R₃, R₄, and R₅ is a halogen atom or a monovalent organic group having a Hammett substituent constant σp of 0.10 or more;
R₆ and R₇ are each independently a hydrogen atom, a halogen atom excluding a fluorine atom, or a monovalent organic group, and one or more of R₆ and R₇ is a halogen atom or a monovalent organic group having a Hammett substituent constant σp of 0.10 or more; and
any adjacent members among R₁, R₂, R₃, R₄, R₅, R₆, and R₇ optionally constitute a portion of a condensed aliphatic ring or a condensed aromatic ring, and the condensed aliphatic ring and the condensed aromatic ring each optionally comprise one or more atoms other than a carbon atom.

2. The compound according to claim 1, wherein
R₁, R₂, R₃, R₄, and R₅ are each independently selected from the group consisting of a hydrogen atom, a halogen atom, a hydroxy group, a thiol group, an amino group, a cyano group, a carboxy group, a nitro group, and an optionally substituted linear, branched or cyclic alkyl group, thioalkyl group, thioaryl group, arylsulfonyl group, aryloxy group, alkylsulfonyl group, alkylamino group, arylamino group, alkoxy group, acylamino group, acyloxy group, aryl group, carboxyamide group, carboalkoxy group, carboaryloxy group, acyl group, and monovalent heterocyclic group, and
R₆ and R₇ are each independently selected from the group consisting of a hydrogen atom, a halogen atom excluding a fluorine atom, a hydroxy group, a thiol group, an amino group, a cyano group, a carboxy group, a nitro group, and an optionally substituted linear, branched or cyclic alkyl group, thioalkyl group, thioaryl group, arylsulfonyl group, aryloxy group, alkylsulfonyl group, alkylamino group, arylamino group, alkoxy group, acylamino group, acyloxy group, aryl group, carboxyamide group, carboalkoxy group, carboaryloxy group, acyl group, and monovalent heterocyclic group.

3. The compound according to claim 1, wherein R₁ is a cyano group.

4. The compound according to claim 1, wherein R₄ and R₅ are hydrogen atoms.

5. The compound according to claim 1, wherein n is 0 and R₆ and R₇ are cyano groups.

6. A compound represented by the following formula (V): 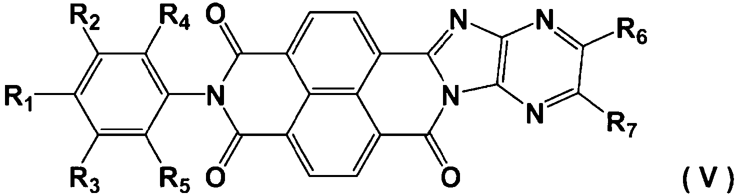 wherein
R₁, R₂, R₃, R₄, and R₅ are each independently selected from the group consisting of a hydrogen atom, a halogen atom, a hydroxy group, a thiol group, an amino group, a cyano group, a carboxy group, a nitro group, and an optionally substituted linear, branched or cyclic alkyl group, thioalkyl group, thioaryl group, arylsulfonyl group, aryloxy group, alkylsulfonyl group, alkylamino group, arylamino group, alkoxy group, acylamino group, acyloxy group, aryl group, carboxyamide group, carboalkoxy group, carboaryloxy group, acyl group, and monovalent heterocyclic group, and one or more of R₁, R₂, R₃, R₄, and R₅ is a hydrogen atom, or a halogen atom or a monovalent organic group having a Hammett substituent constant σp of 0.10 or more; and
R₆ and R₇ are each independently a hydrogen atom, a halogen atom, or a monovalent organic group, one or more of R₆ and R₇ is a halogen atom or a monovalent organic group having a Hammett substituent constant σp of 0.10 or more, any adjacent members among R₁, R₂, R₃, R₄, R₅, R₆, and R₇ optionally constitute a portion of a condensed aliphatic ring or a condensed aromatic ring, and the condensed aliphatic ring and the condensed aromatic ring each optionally comprise one or more atoms other than a carbon atom.

7. The compound according to any one of claims 1 to 6, wherein an energy level of the lowest unoccupied molecular orbital obtained by density functional formalism is -6.00 eV or more and -3.60 eV or less.

8. The compound according to any one of claims 1 to 6, wherein a difference between an energy level of the lowest unoccupied molecular orbital and an energy level of the highest occupied molecular orbital obtained by density functional formalism is 2.85 eV or more and 4.00 eV or less.

9. The compound according to any one of claims 1 to 6, wherein a 5% mass reduction temperature measured under the following Condition 1 is 430°C or higher and 550°C or lower:
(Condition 1)
Pressure: 101325 Pa
Nitrogen: 200 mL/min
Temperature increase condition: increased from 20°C to 550°C at 10°C/min.

10. The compound according to any one of claims 1 to 5, wherein a 10% mass reduction temperature measured under the following Condition 2 is 200°C or higher and 400°C or lower:
(Condition 2)
Pressure: 1 Pa
Temperature increase condition: increased from 20°C to 450°C at 10°C/min.

11. The compound according to any one of claims 1 to 6, wherein the compound is a material for a photoelectric conversion element.

12. An organic thin film comprising the compound according to any one of claims 1 to 6.

13. A photoelectric conversion element comprising a first electrode film, a second electrode film, and a photoelectric conversion film positioned between the first electrode film and the second electrode film, wherein
the photoelectric conversion film comprises the material for a photoelectric conversion element according to claim 11.

14. A photoelectric conversion element comprising a first electrode film, a second electrode film, and a photoelectric conversion film positioned between the first electrode film and the second electrode film, wherein
the photoelectric conversion film comprises the organic thin film according to claim 12.

15. The photoelectric conversion element according to claim 14, wherein
the photoelectric conversion film comprises a photoelectric conversion layer and an auxiliary layer, wherein
the auxiliary layer is made of only the organic thin film or made of a plurality of films comprising the organic thin film.

16. A photoelectric conversion element comprising a first electrode film, a second electrode film, and a photoelectric conversion film positioned between the first electrode film and the second electrode film, wherein
the photoelectric conversion film comprises a photoelectric conversion layer and two auxiliary layers positioned between the photoelectric conversion layer and the second electrode film, wherein
among the two auxiliary layers, an auxiliary layer closer to the second electrode film comprises the organic thin film according to claim 12.

17. An image sensor comprising the photoelectric conversion element according to claim 13.

18. The image sensor according to claim 17, wherein the image sensor is prepared by laminating two or more photoelectric conversion elements.

19. An image sensor prepared by disposing a plurality of photoelectric conversion elements according to claim 13 in an array pattern.

20. A photosensor comprising the image sensor according to claim 17.

21. A solid-state imaging apparatus comprising the image sensor according to claim 17.

22. An image sensor comprising the compound represented by the following formula (VI): 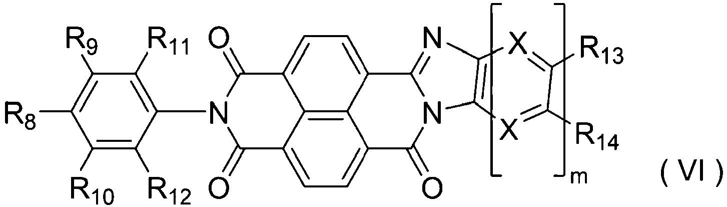 wherein
X is each independently selected from the group consisting of a methine group and a nitrogen atom;
m is an integer of 0 or more and 3 or less;
R₈, R₉, R₁₀, R₁₁, and R₁₂ are each independently a hydrogen atom, a halogen atom having a Hammett substituent constant σp of less than 0.10, or a monovalent organic group having a Hammett substituent constant σp of less than 0.10;
R₁₃ and R₁₄ are each independently a hydrogen atom, a halogen atom, or a monovalent organic group, and one or more of R₁₃ and R₁₄ is a halogen atom or a monovalent organic group having a Hammett substituent constant σp of 0.10 or more; and
any adjacent members among R₈, R₉, R₁₀, R₁₁, R₁₂, R₁₃, and R₁₄ optionally constitute a portion of a condensed aliphatic ring or a condensed aromatic ring, and the condensed aliphatic ring and the condensed aromatic ring each optionally comprise one or more atoms other than a carbon atom.

23. An image sensor comprising a photoelectric conversion element, wherein
the photoelectric conversion element comprises a first electrode film, a second electrode film, and a photoelectric conversion film positioned between the first electrode film and the second electrode film, wherein
the photoelectric conversion film comprises a compound represented by the following formula (VI): 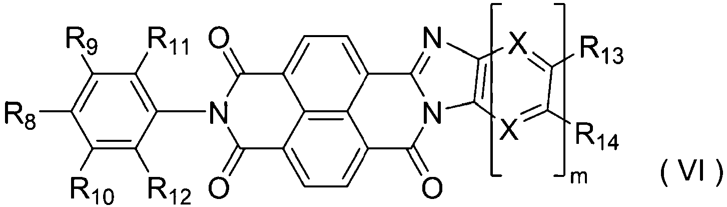
wherein
X is each independently selected from the group consisting of a methine group and a nitrogen atom;
m is an integer of 0 or more and 3 or less;
R₈, R₉, R₁₀, R₁₁, and R₁₂ are each independently a hydrogen atom, a halogen atom having a Hammett substituent constant σp of less than 0.10, or a monovalent organic group having a Hammett substituent constant σp of less than 0.10;
R₁₃ and R₁₄ are each independently a hydrogen atom, a halogen atom, or a monovalent organic group, and one or more of R₁₃ and R₁₄ is a halogen atom or a monovalent organic group having a Hammett substituent constant σp of 0.10 or more; and
any adjacent members among R₈, R₉, R₁₀, R₁₁, R₁₂, R₁₃, and R₁₄ optionally constitute a portion of a condensed aliphatic ring or a condensed aromatic ring, and the condensed aliphatic ring and the condensed aromatic ring each optionally comprise one or more atoms other than a carbon atom.

24. An image sensor comprising a photoelectric conversion element, wherein
the photoelectric conversion element comprises a first electrode film, a second electrode film, and a photoelectric conversion film positioned between the first electrode film and the second electrode film, wherein
the photoelectric conversion film comprises a photoelectric conversion layer and two auxiliary layers positioned between the photoelectric conversion layer and the second electrode film, wherein
among the two auxiliary layers, an auxiliary layer closer to the second electrode film comprises a compound represented by the following formula (VI): 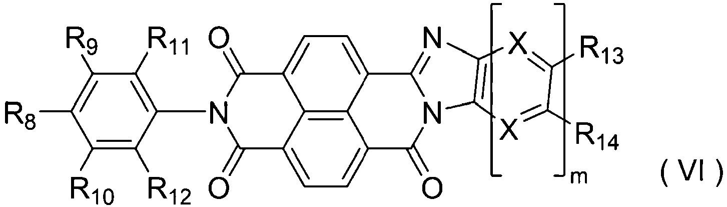
wherein
X is each independently selected from the group consisting of a methine group and a nitrogen atom;
m is an integer of 0 or more and 3 or less;
R₈, R₉, R₁₀, R₁₁, and R₁₂ are each independently a hydrogen atom, a halogen atom having a Hammett substituent constant σp of less than 0.10, or a monovalent organic group having a Hammett substituent constant σp of less than 0.10;
R₁₃ and R₁₄ are each independently a hydrogen atom, a halogen atom, or a monovalent organic group, and one or more of R₁₃ and R₁₄ is a halogen atom or a monovalent organic group having a Hammett substituent constant σp of 0.10 or more; and
any adjacent members among R₈, R₉, R₁₀, R₁₁, R₁₂, R₁₃, and R₁₄ optionally constitute a portion of a condensed aliphatic ring or a condensed aromatic ring, and the condensed aliphatic ring and the condensed aromatic ring each optionally comprise one or more atoms other than a carbon atom.

25. The image sensor according to claim 23 or 24, wherein the image sensor is prepared by laminating two or more photoelectric conversion elements.

26. The image sensor according to claim 23 or 24, wherein the image sensor is prepared by disposing a plurality of photoelectric conversion elements in an array pattern.

27. A photosensor comprising the image sensor according to any one of claims 22 to 24.

28. A solid-state imaging apparatus comprising the image sensor according to any one of claims 22 to 24.
